(19) Europäisches Patentamt
European Patent Office
Office européen des brevets

(11) Veröffentlichungsnummer: **0 164 619**
**B1**

(12) # EUROPÄISCHE PATENTSCHRIFT

(45) Veröffentlichungstag der Patentschrift: **12.10.88**

(21) Anmeldenummer: **85106251.3**

(22) Anmeldetag: **22.05.85**

(51) Int. Cl.[4]: **C 07 C 63/70,** C 07 C 51/363, C 07 C 51/62, C 07 C 121/50, C 07 C 103/22, C 07 C 33/46, C 07 C 25/13, C 07 C 47/24 // A61K31/235

(54) **2,4,5-Trihalogen- bzw. 2,3,4,5-Tetrahalogenbenzolderivate und Verfahren zu ihrer Herstellung.**

(30) Priorität: **04.06.84 DE 3420796**

(43) Veröffentlichungstag der Anmeldung: **18.12.85 Patentblatt 85/51**

(45) Bekanntmachung des Hinweises auf die Patenterteilung: **12.10.88 Patentblatt 88/41**

(84) Benannte Vertragsstaaten: **AT BE CH DE FR GB IT LI NL SE**

(56) Entgegenhaltungen:
**US-A-3 290 353**
**US-A-3 816 526**

**CHEMICAL ABSTRACTS, Band 100, Nr. 7, 13. Februar 1984, Seite 553, Nr. 51279p, Columbus, Ohio, US; & JP - A - 58 150 543 (CHISSO CORP.) 07.09.1983**

(73) Patentinhaber: **BAYER AG, Konzernverwaltung RP Patentabteilung, D-5090 Leverkusen 1 Bayerwerk (DE)**

(72) Erfinder: **Klauke, Erich, Dr., Eichendorffweg 8, D-5068 Odenthal (DE)**
Erfinder: **Petersen, Uwe, Dr., Auf dem Forst 4, D-5090 Leverkusen 1 (DE)**
Erfinder: **Grohe, Klaus, Dr., Am Wasserturm 10, D-5068 Odenthal (DE)**

**EP 0 164 619 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft 2,4,5-Trihalogen- bzw. 2,3,4,5-Tetrahalogenbenzolderivate und Verfahren zu ihrer Herstellung. Die erfindungsgemäßen Verbindungen sind wertvolle Zwischenprodukte für die Synthese hochwirksamer antibakterieller Arzneimittel.

Gefunden wurden 2,4,5-Trihalogen- bzw. 2,3,4,5-Tetrahalogenbenzolderivate der Formel (I)

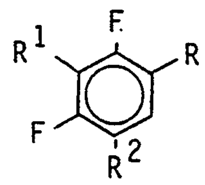

(I)

in welcher

R für -COOH, -COCl, -COF, -CN, -$CONH_2$, -$CH_2OH$-$CH_2Cl$, -$CHCl_2$, -$CCl_3$ oder -CHO,

$R^1$ für Cl oder F und

$R^2$ für Cl oder F stehen,

wobei nur $R^1$ oder $R^2$ F sein können.

Insbesondere seinen erwähnt:

2,4,5-Trifluorbenzoylfluorid;

2,3,4-Trifluor-5-chlorbenzoylfluorid;

2,4,5-Trifluor-3-chlorbenzoylchlorid;

2,4-Difluor-3,5-dichlorbenzoylfluorid.

Weiterhin wurde gefunden, daß man die 2,4,5-Trihalogen- bzw. 2,3,4,5-Tetrahalogenbenzolderivate der Formel (I)

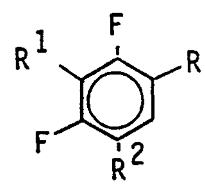

(I)

in welcher

R für -COOH-COCl, -COF, -CN, -$CONH_2$, -$CH_2OH$, -$CH_2Cl$, -$CHCl_2$, -$CCl_3$ oder -CHO,

$R^1$ für Cl oder F und

$R^2$ für Cl oder F stehen,

wobei nur $R^1$ oder $R^2$ F sein können,

dadurch erhalten kann, daß man 2,3,4,5-Tetrachlorbenzonitril mit Kaliumfluorid in einem Lösungsmittel bei erhöhter Temperatur umsetzt und die erhaltenen Nitrile in an sich bekannter Weise in die Verbindungen der Formel (I) überführt.

Weiterhin wurde gefunden, daß man 2,3,4,5-Tetrahalogenbenzolderivate der Formel (II)

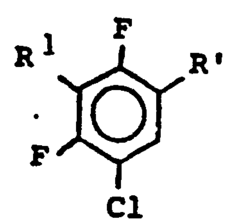

(II)

in welcher

R' für -COCl oder -COF und

$R^1$ für Cl oder F stehen,

dadurch erhalten kann, daß man 2,3,4,5-Tetrachlorbenzoylchlorid oder 2,3,4,5-Tetrachlorbenzoylfluorid, (siehe Europäisches Patent Nr. 57 844), gegebenenfalls nach Fluorierung mit Fluorwasserstoffsäure, mit Kaliumfluorid in einem Lösungsmittel bei erhöhter Temperatur umsetzt.

Die Menge des einzusetzenden Kaliumfluorids richtet sich nach der Anzahl der auszutauschenden Chloratome. Für 1 Chloratom wird mindestens ein Mol KF eingesetzt, im allgemeinen jedoch 1,1-1,5 Mol.

Maximal werden 2 Mol KF/1 Chlor verwendet; darüber hinaus ist die Menge KF praktisch ohne Einfluß auf den Fluorierungsgrad und das Verfahren wird unökonomisch. Es läßt sich jedoch ein Teil des teuren KF einsparen, wenn man zuvor das 2,3,4,5-Tetrachlorbenzoylchlorid mit Fluorwasserstoffsäure fluoriert und das dabei in praktisch quantitativer Ausbeute anfallende 2,3,4,5-Tetrachlorbenzoylfluorid zur Cl/F-Austauschreaktion mit KF einsetzt. Wegen der stärkeren Aktivierung durch die elektronegativere Fluorcarbonylgruppe, ihrer höheren thermischen Stabilität und die verminderte KCl-Belastung im Reaktionsgemisch, führt diese Zweistufenfluorierung insgesamt zu einer verbesserten Bilanz bei der Kernfluorierung.

Als Lösungsmittel bei der Kernfluorierung können die für Fluorierungsreaktionen bekannten inerten Lösungsmittel, z. B. Dimethylformamid, Dimethylsulfoxid, N-Methylpyrrolidon, Diethylsulfon u.a. verwendet werden. Besonders bevorzugt wird jedoch Tetramethylensulfon (Sulfolan) eingesetzt.

Die Reaktionstemperatur liegt zwischen 180 und 260°C in Abhängigkeit vom gewünschten Fluorierungsgrad. Während bei der niederen Temperatur auch noch kernfluorfreies Produkt gefunden wird, entsteht bei den höheren Temperaturen bereits ein recht deutlicher Anteil des bekannten 2,3,4,5-Tetrafluorbenzoylfluorids (siehe die eigene Deutsche Patentanmeldung P-3 318 145 vom 18.05.83).

Wegen der thermischen Labilität der im Reaktionsgemisch vorhandenen Tetrahalogenbenzoylhalogenide - vorzugsweise Bildung von Octahalogenbenzophenonen - hat es sich als vorteilhaft erwiesen, das gewünschte Reaktionsprodukt während der Fluorierungsreaktion laufend durch Destillation aus dem Reaktionsraum zu entfernen. Das geschieht zweckmäßig über eine Fraktionierkolonne mit einer steten Anpassung des Destillationsdruckes an den Partialdruck des Fluorierungsgemisches.

Weiterhin wurde gefunden, daß man 2,4,5-Trifluor-3-chlorbenzoylchlorid dadurch erhalten kann, daß man 2,4,5-Trifluorbenzoesäurechlorid zur 2,4,5-Trifluor-3-chlorbenzoesäure und diese mit Thionylchlorid umsetzt. Die dafür als Ausgangsprodukt benötigte 2,4,5-Trifluorbenzoesäure ist bereits bekannt [J.I. de Graw, M. Cory, W.A. Skinner, J. Chem. Eng. Data 13, 587 (1968)]. Ihre Herstellung gemäß dieser Literaturstelle erfolgt in einer sehr schlechten Ausbeute aus 2-Amino-4,5-difluorbenzoesäureester durch $NH_2$/F-Austausch nach Art einer Balz-Schiemann-Reaktion. Aus DE-OS-3 142 856 = US-PS-4 439 620 ist jedoch das 2,4-Dichlor-5-fluor-benzoylchlorid bekannt geworden. Es wurde nun weiterhin gefunden, daß man daraus in einfacher Weise durch Fluorierung mit KF in einem Lösungsmittel, vorzugsweise Tetramethylensulfon, bei Temperaturen von 180 bis 230°C das neue 2,4,5-Trifluorbenzoylfluorid in guter Ausbeute erhält, welches durch alkalische Hydrolyse praktisch quantitativ in 2,4,5-Trifluorbenzoesäure überführt wird.

Die Chlorierung der 2,4,5-Trifluorbenzoesäure erfolgt in der Schmelze unter Druck und/oder in einem Lösungsmittel, z. B. Chlorsulfonsäure oder Oleum, in Gegenwart von Halogenüberträgern, z. B. Jod. Man erhält dabei die 2,4,5-Trifluor-3-chlorbenzoesäure. Da das Reaktionsgemisch jedoch noch unverändertes Ausgangsmaterial und etwas 2,4,5-Trifluor-3,6-dichlorbenzoesäure enthält, wird der Rohansatz ohne Zwischenisolierung mit Thionylchlorid behandelt. Man erhält dann durch fraktionierte Destillation das gewünschte 2,4,5-Trifluor-3-chlorbenzoylchlorid. Günstiger ist es jedoch, die destillative Trennung über die Säurefluoride durchzuführen.

Die Funktionalisierung der Nitrile in die entsprechenden Säurehalogenide erfolgt z. B. Hydrolyse zur Carbonsäure und Überführung in Säurechloride mit Thionylchlorid. Aus den Säurechloriden erhält man dann die Säurefluoride durch Reaktion mit Fluorierungsmitteln, z. B. wasserfreier Fluorwasserstoffsäure oder Alkalifluoriden. Will man umgekehrt aus einem Säurefluorid das Säurechlorid herstellen, dann geschieht dies auf elegante Weise durch Reaktion mit $SiCl_4$, gegebenenfalls in Gegenwart katalytischer Mengen Aluminiumchlorid. Halogensubstituierte Benzylalkohole lassen sich in glatter Reaktion aus den Säurehalogeniden, besonders gut aus den Säurefluoriden, durch Reduktion mit Natriumboranat herstellen.

Mit Thionylchlorid erhält man aus den halogenierten Benzylalkoholen in praktisch quantitativer Ausbeute die entsprechenden Benzylchloride, die sich mit gasförmigem Chlor stufenweise zu entsprechenden Benzal- und Benzotrichloriden weiterchlorieren lassen.

Durch vorzugsweise saure Hydrolyse der halogenierten Benzalchloride werden die halogenierten Benzaldehyde gewonnen, während die entsprechende Hydrolyse der halogenierten Benzotrichloride sowohl entsprechende Säurechloride als auch Säuren ergibt.

Die erfindungsgemäßen Verbindungen sollen als Ausgangsprodukte für die Synthese von Arzneimitteln verwendet werden.

Die erfindungsgemäßen Verbindungen können beispielsweise in hochwirksame antibakterielle 1-Cyclopropyl-6,8-dihalogen-1,4-dihydro-4-oxo-7-(1-piperazinyl)-3-chinolincarbonsäuren überführt werden. Die Synthese kann z. B. nach folgendem Schema unter Verwendung von 5-Chlor-2,3,4-trifluorbenzoylfluorid als Ausgangsprodukt durchgeführt werden.

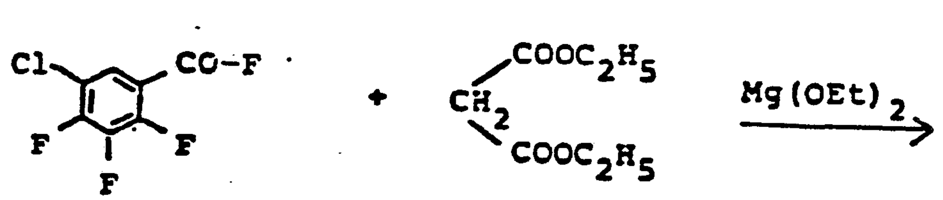

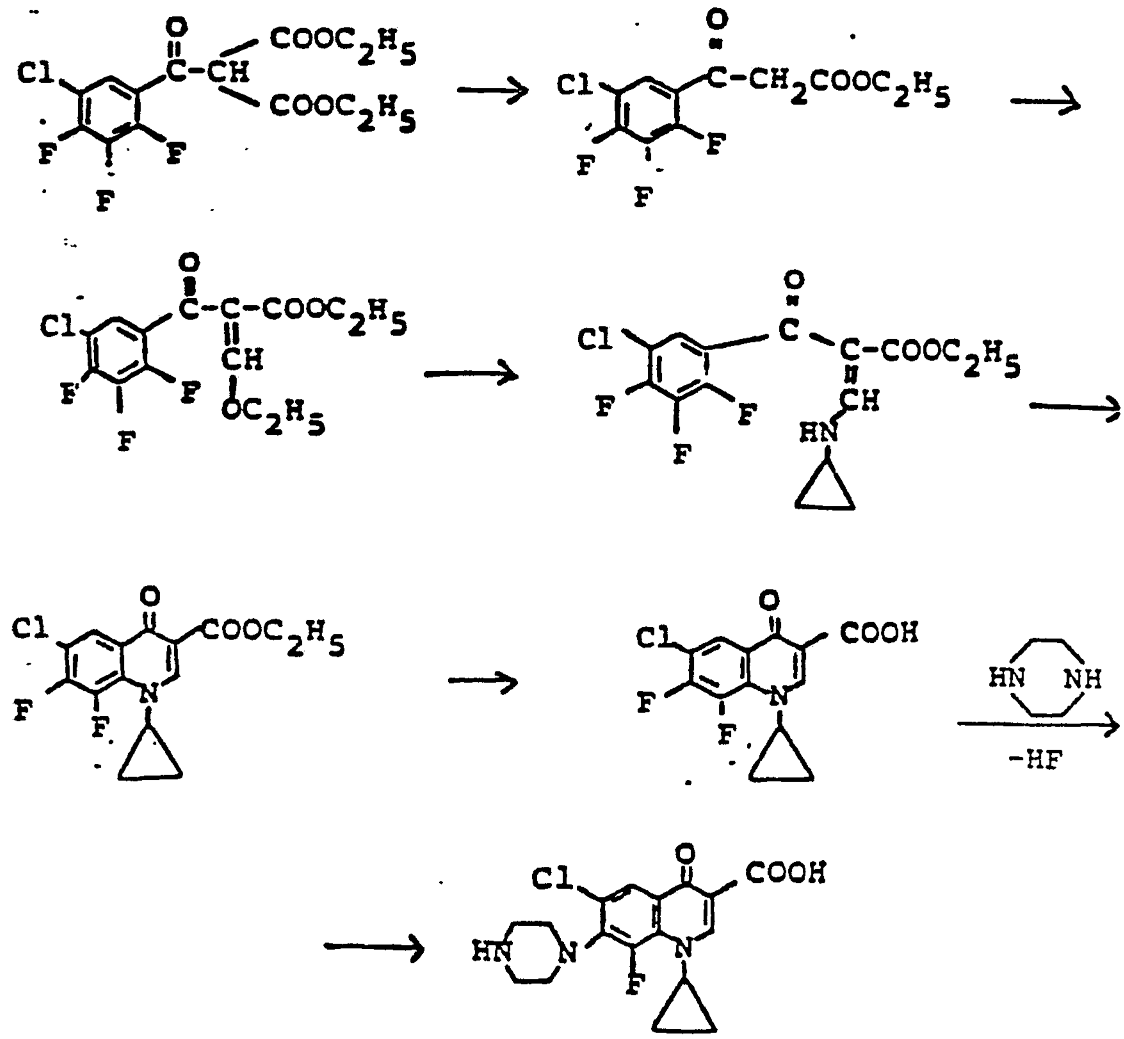


Insbesondere können mit den erfindungsgemäßen Verbindungen 7-Amino-1-cyclopropyl-6,8-dihalogen-1,4-dihydro-4-oxo-3-chinolincarbonsäuren der Formel (I')

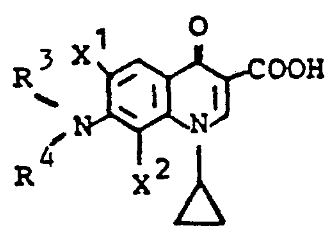

(I')

erhalten werden,
in welcher

- $X^1$ und $X^2$ gleich oder verschieden sein können und für Chlor oder Fluor stehen, jedoch nicht gleichzeitig Fluor sein können und
- $R^3$ und $R^4$ gemeinsam mit dem Stickstoffatom, an das sie gebunden sind, einen 5- oder 6-gliedrigen heterocyclischen Ring bilden, der als Ringglied zusätzlich die Atome oder Gruppen -O-, -S-, -SO-, $-SO_2-$, $>N-R^5$ oder $-CO-N-R^5$ enthalten kann und der gegebenenfalls an den Kohlenstoffatomen ein- bis dreifach durch $C_1$-$C_4$-Alkyl, gegebenenfalls durch Chlor, Fluor, Brom, Methyl, Phenyl, Hydroxy, Methoxy, Benzyloxy, Nitro oder Piperidino ein- bis dreifach substituiertes Phenyl oder Cyclohexyl, 2-Thienyl, Hydroxy, Alkoxy mit 1 bis 3 Kohlenstoffatomen, Amino, Methylamino oder Ethylamino substituiert sein kann, wobei
- $R^5$ für Wasserstoff, eine verzweigte oder unverzweigte Alkyl-, Alkenyl- oder Alkinylgruppe mit 1 bis 6 Kohlenstoffatomen, die gegebenenfalls durch eine oder zwei Hydroxy-, Alkoxy-, Alkylamino- oder

Dialkylaminogruppen mit 1 bis 3 Kohlenstoffatomen für einen Alkylrest, die Cyangruppe, die Alkoxycarbonylgruppe mit 1 bis 4 Kohlenstoffatomen im Alkoholteil substituiert sein kann, eine gegebenenfalls im Phenylrest substituierte Phenylalkylgruppe mit bis zu 4 Kohlenstoffatomen im aliphatischen Teil, einen gegebenenfalls durch Hydroxy, Methoxy, Chlor oder Fluor ein- oder zweifach substituierten Phenacylrest, einen Oxoalkylrest mit bis zu 6 Kohlenstoffatomen steht, ferner einen Rest $COR^6$, CN oder $SO_2R^7$ bedeutet,

$R^6$ Wasserstoff, gegebenenfalls durch 1 oder 2 Substituenten aus der Reihe Amino, Alkoxycarbonyl mit 1 bis 3 Kohlenstoffatomen im Alkylteil, Carboxy oder Alkoxy mit 1 bis 3 Kohlenstoffatomen oder Halogen wie Chlor, Brom, Fluor substituiertes geradkettiges oder verzweigtes Alkyl mit 1 bis 4 Kohlenstoffatomen, Alkoxy mit 1 bis 4 Kohlenstoffatomen, Amino, Alkylamino oder Dialkylamino mit 1 bis 5 Kohlenstoffatomen im Alkylteil und

$R^7$ geradkettiges oder verzweigtes Alkyl mit 1 bis 3 Kohlenstoffatomen darstellt, und deren pharmazeutisch verwendbaren Hydrate, Säureadditionssalze, Alkali-, Erdalkali- und Guanidiniumsalze, die eine hohe antibakterielle Wirkung aufweisen.

Sie eignen sich daher als Wirkstoffe für die Human- und Veterinärmedizin, wobei zur Veterinärmedizin auch die Behandlung von Fischen zur Therapie oder Vorbeugung bakterieller Infektionen zu zählen ist.

Bevorzugt werden diejenigen Verbindungen der Formel (I') erhalten, in denen

$X^1$ und $X^2$ gleich oder verschieden sein können und für Chlor oder Fluor stehen, jedoch nicht gleichzeitig Fluor sein können und

$R^3$ und $R^4$ gemeinsam mit dem Stickstoffatom, an das sie gebunden sind, einen 5- oder 6-gliedrigen heterocyclischen Ring bilden können, der als Ringglied zusätzlich die Atome oder Gruppen -O-, -S-, $-SO_2-$, $N-R^5$ oder $-CO-N-R^5$ enthalten kann und der gegebenenfalls an den Kohlenstoffatomen ein- bis zweifach durch $C_1$-$C_3$-Alkyl, Cyclohexyl, gegebenenfalls durch Chlor, Fluor, Brom, Methyl, Phenyl, Hydroxy, Methoxy, Benzyloxy, Nitro oder Piperidino ein oder zweifach substituiertes Phenyl, 2-Thienyl, Hydroxy, Amino oder Methylamino substituiert sein kann, wobei

$R^5$ für Wasserstoff, eine verzweigte oder unverzweigte Alkyl-, Alkenyl- oder Alkinylgruppe mit 1 bis 4 Kohlenstoffatomen, die gegebenenfalls durch eine oder zwei Hydroxygruppen substituiert sein kann, einen Phenacylrest, einen Oxoalkylrest mit bis zu 5 Kohlenstoffatomen sowie für einen Rest $COR^6$, steht, wobei

$R^6$ Wasserstoff, geradkettiges oder verzweigtes Alkyl mit 1 bis 3 Kohlenstoffatomen, Alkoxy mit 1 bis 3 Kohlenstoffatomen, Amino, Alkylamino oder Dialkylamino mit 1 bis 3 Kohlenstoffatomen im Alkylteil bedeutet.

Besonders bevorzugt werden diejenigen Verbindungen der Formel (I') erhalten, in denen

$X^1$ und $X^2$ gleich oder verschieden sein können und für Chlor oder Fluor stehen, jedoch nicht gleichzeitig Fluor sein können und

$R^3$ und $R^4$ gemeinsam mit dem Stickstoffatom, an das sie gebunden sind, einen 5- oder 6-gliedrigen heterocyclischen Ring bilden können, der als Ringglied zusätzlich ein Sauerstoffatom oder die Gruppen $N-R^5$ oder $-CO-N-R^5$ enthalten kann und der gegebenenfalls an den Kohlenstoffatomen ein- bis zweifach durch $C_1$-$C_2$-Alkyl, Cyclohexyl, gegebenenfalls durch Chlor, Fluor, Methyl, Phenyl, Hydroxy, Methoxy, Benzyloxy, Nitro oder Piperidino substituiertes Phenyl, 2-Thienyl oder Hydroxy substituiert sein kann, wobei

$R^5$ für Wasserstoff, eine verzweigte oder unverzweigte Alkylgruppe mit 1 bis 3 Kohlenstoffatomen, die gegebenenfalls durch eine oder zwei Hydroxygruppen substituiert sein kann, einen Phenacylrest, einen Oxoalkylrest mit bis zu 4 Kohlenstoffatomen sowie einen Rest $COR^6$ steht, wobei

$R^6$ Wasserstoff oder Alkyl mit ein oder zwei Kohlenstoffatomen bedeutet.

Die Verbindungen der Formel (I') werden erhalten, wenn man die 1-Cyclopropyl-7-halogen-1,4-dihydro-4-oxo-3-chinolincarbonsäuren der Formel (II'),

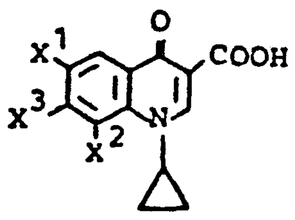

(II')

in welcher

$X^1$ und $X^2$ die oben angegebene Bedeutung haben, und

$X^3$ für Halogen, bevorzugt Chlor oder Fluor, steht, mit Aminen der Formel (III),

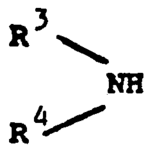

(III)

in welcher
$R^3$ und $R^4$ die oben angegebene Bedeutung haben,
gegebenenfalls in Gegenwart von Säurebindern umsetzt (Methode A).

Die Verbindungen der Formel (I') können auch erhalten werden, indem man eine 7-(1-Piperazinyl)-3-chinoloncarbonsäure der Formel (IV),

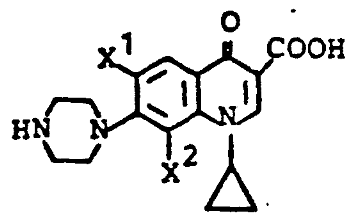

(IV)

in welcher
$X^1$ und $X^2$ die oben angegebene Bedeutung haben und
der Piperazinylrest an den Kohlenstoffatomen 1-3 fach durch $C_1$-$C_4$-Alkyl, 2-Thienyl oder gegebenenfalls substituiertes Cyclohexyl oder Phenyl substituiert sein kann, mit Verbindungen der Formel (V)

$R^5$ (V)

in welcher
$R^5$ die oben angegebene Bedeutung hat, jedoch nicht Wasserstoff sein kann, und
X Fluor, Chlor, Brom, Iod, Hydroxy, Acyloxy, Ethoxy, Phenoxy, 4-Nitrophenoxy bedeutet,
gegebenenfalls in Gegenwart von Säurebindern umsetzt (Methode B).

Man erhält die Verbindungen der Formel (I') auch, wenn man eine 7-(1-Piperazinyl)-3-chinoloncarbonsäure der Formel (IV), in welcher der Piperazinylrest an den Kohlenstoffatomen 1-3-fach durch $C_1$-$C_4$-Alkyl, 2-Thienyl oder gegebenenfalls substituiertes Cyclohexyl oder Phenyl substituiert sein kann, mit Michael-Acceptoren der Formel (VI),

$B\text{-}CH{=}CH_2$ (VI)

in der B für CN, CO-$R^8$ oder COO$R^9$ steht,
wobei $R^8$ für Methyl oder Ethyl und
$R^9$ für Methyl, Ethyl, n- oder i-Propyl steht,
umsetzt (Methode C).

Verwendet man bei der Umsetzung nach Methode A 2-Methylpiperazin und 6-Chlor-1-cyclopropyl-7,8-difluor-1,4-dihydro-4-oxo-3-chinolincarbonsäure als Ausgangsstoffe, so kann der Reaktionsablauf durch das folgende Formelschema wiedergegeben werden:

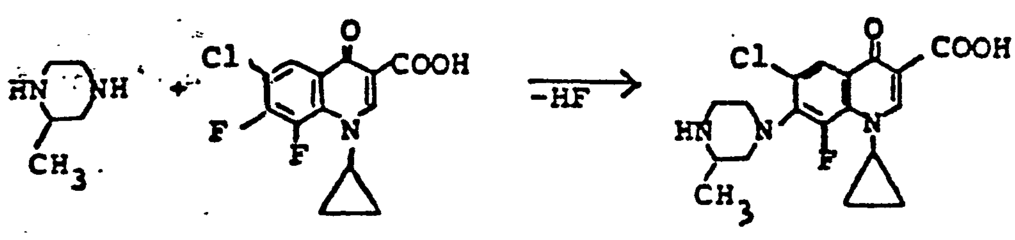


Verwendet man bei der Umsetzung nach Methode B Ethyliodid und 6-Chlor-1-cyclopropyl-8-fluor-1,4-dihydro-4-oxo-7-(1-piperazinyl)-3-chinolincarbonsäure als Ausgangsstoffe, so kann der Reaktionsablauf durch das folgende Formelschema wiedergegeben werden:

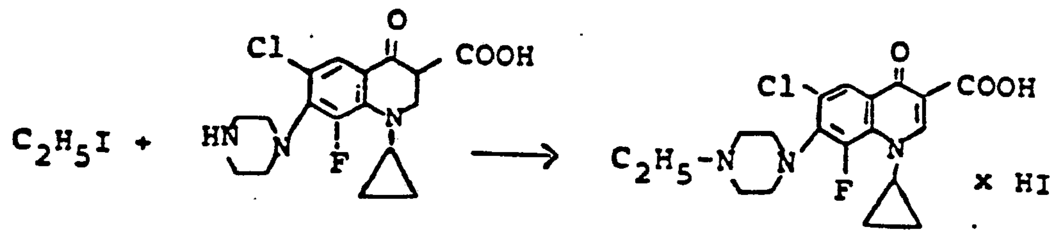

Verwendet man beispielsweise bei der Umsetzung von (IV) mit (V) nach Methode B 6-Chlor-1-cyclopropyl-8-fluor-1,4-dihydro-7-(3-methyl-1-piperazinyl)-4-oxo-3-chinolincarbonsäure und Ameisen-essigsäure-anhydrid als Ausgangsverbindungen, so kann der Reaktionsablauf durch das folgende Formelschema wiedergegeben werden:

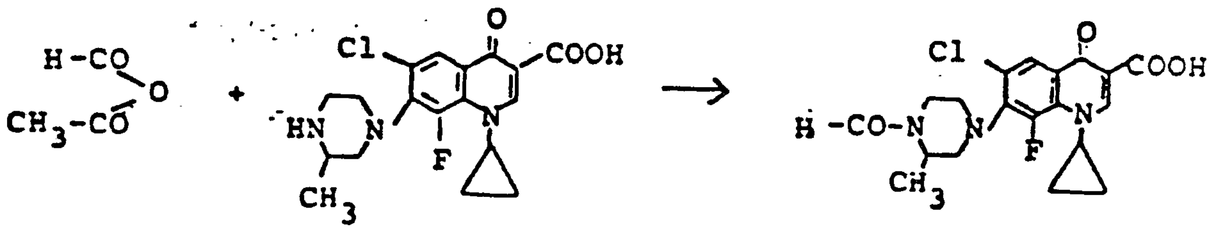

Verwendet man beispielsweise nach Methode C 6-Chlor-1-cyclopropyl-8-fluor-1,4-dihydro-4-oxo-7-(1-piperazinyl)-3-chinolincarbonsäure und Methylvinylketon als Ausgangsverbindungen, so kann der Reaktionsablauf durch das folgende Formelschema wiedergegeben werden:

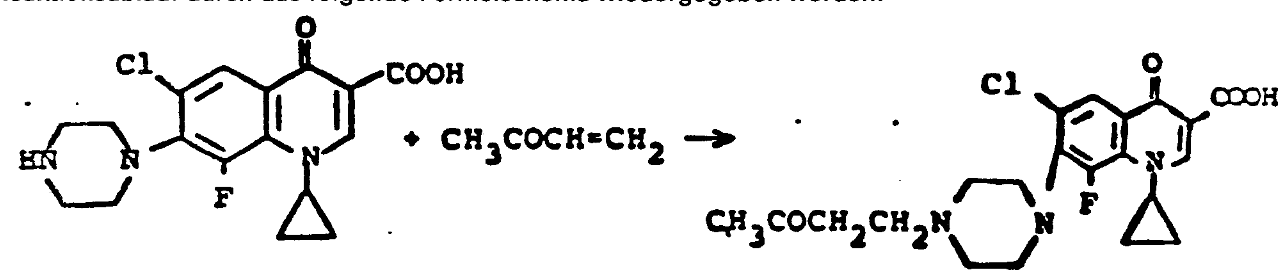

Die als Ausgangsstoffe nach Methode A verwendbaren 1-Cyclopropyl-6,7,8-trihalogen-1,4-dihydro-4-oxo-3-chinolincarbonsäuren der Formel (II') können gemäß folgendem Reaktionsschema hergestellt werden:

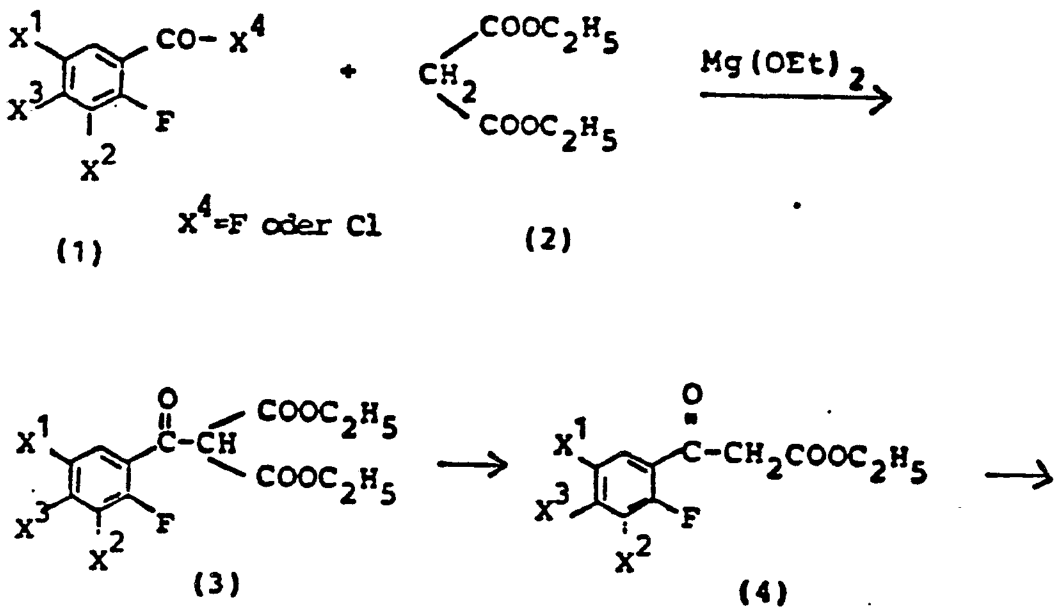


$X^4$=F oder Cl

(1) (2)

(3) (4)

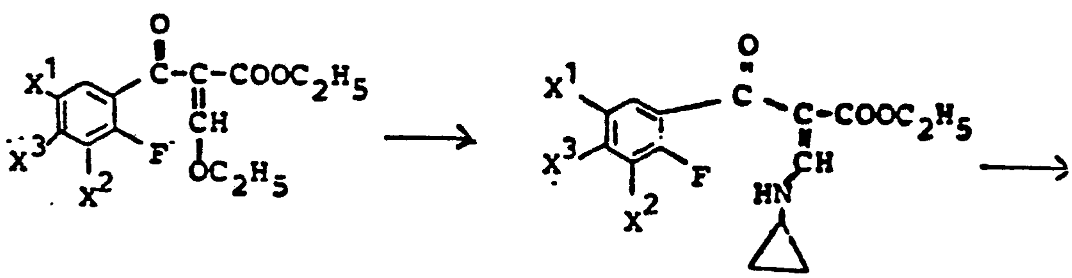

(5) (6)

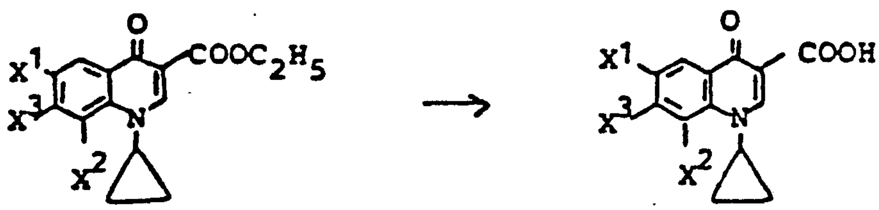

(7) (II')

Danach wird Malonsäurediethylester (2) in Gegenwart von Magnesiumethylat mit dem entsprechenden Benzoylfluorid bzw. -chlorid (1) zum Aroylmalonester (3) acyliert (Organicum, 3. Aufl. 1964, S. 438).

Durch partielle Verseifung und Decarboxylierung von (3) in wäßrigem Medium mit katalytischen Mengen Schwefelsäure oder p-Toluolsulfonsäure erhält man in guter Ausbeute den Aroylessigsäureethylester (4), der mit Orthoameisensäure-triethylester/Acetanhydrid in den 2-(2,3, 4,5-Tetrahalogenbenzoyl)-3-ethoxy-acrylsäureethylester (5) übergeht. Die Umsetzung von (5) mit Cyclopropylamin in einem Lösungsmittel, wie z. B. Methylenchlorid, Alkohol, Chloroform, Cyclohexan oder Toluol führt in leicht exothermer Reaktion zum gewünschten Zwischenprodukt (6).

Die Cyclisierungsreaktion (6) → (7) wird in einem Temperaturbereich von etwa 60 bis 300° C, bevorzugt 80 bis 180° C durchgeführt.

Als Verdünnungsmittel können Dioxan, Dimethylsulfoxid, N-Methylpyrrolidon, Sulfolan, Hexamethylphosphorsäuretrisamid und bevorzugt N,N-Dimethylformamid verwendet werden.

Als Säurebinder kommen für diese Reaktionsstufe Kaliumtert.-butanolat, Butyl-lithium, Lithium-phenyl, Phenylmagnesiumbromid, Natriummethylat, Natriumhydrid, Natrium- oder Kalium-carbonat und besonders bevorzugt Kalium- oder Natrium-fluorid in Betracht. Es kann vorteilhaft sein, einen Überschuß von 10 Mol-% an Base einzusetzen.

Die in dem letzten Schritt erfolgende Esterhydrolyse von (7) unter basischen oder sauren Bedingungen führt zu den 1-Cyclopropyl-6,7,8-trihalogen-1,4-dihydro-4-oxo-3-chinolincarbonsäuren (II').

Die als Ausgangsstoffe für diesen Syntheseweg verwendeten Benzoylhalogenide (1) werden wie folgt hergestellt:

3,5-Dichlor-2,4-difluor-benzoylfluorid (Siedepunkt 97°/20 mbar; $n^{20}_D$ = 1,5148) und 5-Chlor-2,3,4-trifluorbenzoylfluorid (Siedepunkt 68 - 70°/20 mbar; $n^{20}_D$ = 1,4764) erhält man nebeneinander beim Erhitzen von Tetrachlorbenzoylchlorid mit Kaliumfluorid im Sulfolan auf erhöhte Temperaturen:

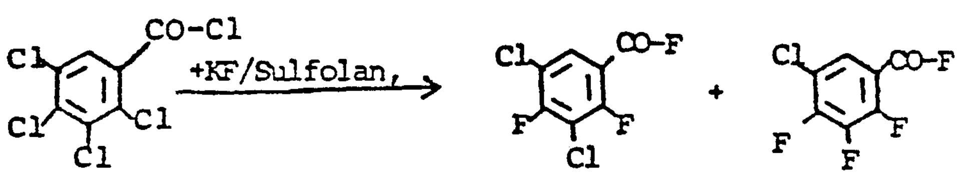


Die Chlorierung von 2,4,5-Trifluorbenzoesäure in Chlorsulfonsäure führt zur 3-Chlor-2,4,5-trifluorbenzoesäure, die als Rohprodukt mit Thionylchlorid zum 3-Chlor-2,4,5-trifluorbenzoylchlorid (Siedepunkt 94°/18 mbar; $n^{20}_D$ = 1,5164) umgesetzt wird:

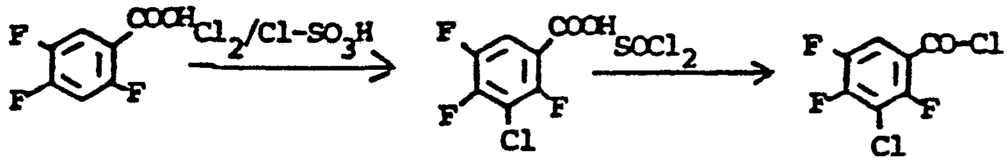


Die als Ausgangsstoffe verwendeten Amine (III) sind bekannt oder können nach literaturbekannten Verfahren erhalten werden [US-4 166 180, J. Med. Chem. 26, 1116 (1983)]. Durch katalytische Hydrierung werden aus den 2-Aryl-piperazinen die entsprechenden 2-Cyclohexylpiperazine erhalten; z. B.: 2-Cyclohexylpiperazin (wachsartig, Schmelzpunkt 71 - 73° C). Als Beispiele seien genannt:

Morpholin, Piperidin, Thiomorpholin, Pyrrolidin, Piperazin, N-Methylpiperazin, N-Ethylpiperazin, N-(2-Hydroxyethyl)-piperazin, N-Formylpiperazin, 2-Methylpiperazin, 1,2-Dimethylpiperazin, cis- und trans-2,5-Dimethylpiperazin, cis- und trans-2,6-Dimethylpiperazin, 2-Ethylpiperazin, 2-Propylpiperazin, 2-Isopropylpiperazin, 2-Isobutylpiperazin, 2-Piperazinon, 1-Methyl-2-piperazinon, 1-Ethyl-2-piperazinon, 2-Cyclohexylpiperazin, 2-Phenylpiperazin, 2-(4-Chlorphenyl)-piperazin, 2-(4-Fluorphenyl)-piperazin, 2-(4-Bromphenyl)-piperazin, 2-(4-Methylphenyl)-piperazin, 2-(4-Biphenylyl)-piperazin, 2-(4-Methoxyphenyl)-piperazin, 2-(4-Benzyloxyphenyl)-piperazin, 2-(4-Hydroxyphenyl)-piperazin, 2-(4-Nitrophenyl)-piperazin, 2-(3-Nitrophenyl)-piperazin, 2-(4-Piperidinophenyl)-piperazin, 2-(3,4-Dimethoxyphenyl)-piperazin, 2-(3,4,5-Trimethoxyphenyl)-piperazin, 2-(3,4-dimethoxy-6-methyl)-piperazin, 2-(2-Thienyl)-piperazin, 3-Aminopyrrolidin.

Die als Ausgangsstoffe verwendeten Verbindungen der Formel (V) sind bekannt. Als Beispiele seinen genannt:

Methyliodid, Methylbromid, Ethyliodid, Ethylbromid, Ethylchlorid, 2-Hydroxyethylchlorid, 3-Hydroxypropylchlorid, 4-Hydroxybutylchlorid, n-Propylbromid, i-Propyliodid, n-Butylbromid, i-Butylbromid, sek.-Butylchlorid, n-Pentylchlorid, 3-Methylbutylchlorid, n-Hexylbromid, Ameisensäureessigsäureanhydrid, Essigsäureanhydrid, Propionsäureanhydrid, Acetylchlorid, Chloracetylchlorid, Dichloracetylchlorid, Bromacetylbromid, Buttersäurechlorid, 4-Chlorbuttersäurechlorid, Isobuttersäurechlorid, N-(tert.-Butoxycarbonyl)-glycin-4-nitrophenylester, N-(tert.-Butoxycarbonyl)-L-alanin-4-nitro-phenylester, N-(tert.-Butoxycarbonyl)-L-leucin-4-nitrophenylester, N-(tert.-Butoxycarbonyl)-L-valin-4-nitrophenylester, 3-Methoxypropionsäurechlorid, Chlorkohlensäuremethylester, Chlorkohlensäureethylester, Chlorkohlensäure-n-butylester, Diethylcarbonat, Chlorcyan, Diphenylcarbonat, Bromcyan, Dimethylcarbamidsäurechlorid, Methansulfonsäurechlorid, Ethansulfonsäurechlorid, Propan-1-sulfonsäurechlorid, Ameisensäure.

Die erfindungsgemäß verwendbaren Verbindungen der Formel (VII) sind bekannt. Als Beispiele seien genannt:

Acrylnitril, Methylvinylketon, Acrylsäuremethylester, Acrylsäureethylester.

Die Umsetzung von (II') mit (III) gemäß Methode A wird vorzugsweise in einem Verdünnungsmittel wie Dimethylsulfoxid, N,N-Dimethylformamid, Hexamethyl-phosphorsäuretrisamid, Sulfolan, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diaza-bicyclo[2,2,2]-octan (DABCO), 1,8-Diaza-bicyclo[5,4,0]-undec-7-en (DBU) oder überschüssiges Amin (III).

Die Reaktionstemperaturen können in einem größeren Bereich variiert werden. Im allgemeinen arbeitet man zwischen etwa 20 und 200° C, vorzugsweise zwischen 80 und 180° C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens setzt man auf 1 Mol der Carbonsäure (II') 1 bis 15 Mol, vorzugsweise 1 bis 6 Mol des Amins (III) ein.

Die Umsetzung von (IV) mit (V) wird vorzugsweise in einem Verdünnungsmittel wie Dimethylsulfoxid, Dioxan, N,N-Dimethylformamid, Hexamethyl-phosphorsäure-trisamid, Sulfolan, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2,2,2]octan (DABCO) oder 1,8-Diazabicyclo-[5,4,0]undec-7-en (DBU).

Die Reaktionstemperaturen können in einem größeren Bereich variiert werden. Im allgemeinen arbeitet man zwischen etwa 20 und etwa 180° C, vorzugsweise zwischen 40 und 110° C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens gemäß Methode B setzt man auf 1 Mol der Verbindung (IV) 1 bis 4 Mol, vorzugsweise 1 bis 1,5 Mol der Verbindung (V) ein.

Die Umsetzung von (IV) mit (VI) (Methode C) wird vorzugsweise in einem Verdünnungsmittel wie Dioxan, Dimethylsulfoxid, N,N-Dimethylformamid, Methanol, Ethanol, Isopropanol, n-Propanol, Glykolmonomethylether oder auch in Gemischen dieser Verdünnungsmittel durchgeführt.

Die Reaktionstemperaturen können in einem größeren Bereich variiert werden. Im allgemeinen arbeitet man zwischen etwa 20° C und etwa 150° C, vorzugsweise zwischen 50° C und 100° C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens nach Methode C setzt man auf 1 Mol der Verbindung (IV) 1 bis 5 Mol, vorzugsweise 1 bis 2 Mol, der Verbindung (VI) ein.

Außer den in den Beispielen aufgeführten Verbindungen seien als neue Wirkstoffe im einzelnen genannt:

6-Chlor-7-[3-(4-chlorphenyl)-1-piperazinyl]-1-cyclopropyl-8-fluor-1,4-dihydro-4-oxo-3-chinolincarbonsäure,
6-Chlor-1-cyclopropyl-8-fluor-7-[3-(4-fluorphenyl)-1-piperazinyl]-1,4-dihydro-4-oxo-3-chinolincarbonsäure,
7-[3-(4-Bromphenyl)-1-piperazinyl]-6-chlor-1-cyclopropyl-8-fluor-1,4-dihydro-4-oxo-3-chinolincarbonsäure,
6-Chlor-1-cyclopropyl-8-fluor-1,4-dihydro-7-[3-(4-methylphenyl)-1-piperazinyl]-4-oxo-3-chinolincarbonsäure,
7-[3-(4-Biphenylyl)-1-piperazinyl]-6-chlor-1-cyclopropyl-8-fluor-1,4-dihydro-4-oxo-3-chinolincarbonsäure,
6-Chlor-1-cyclopropyl-8-fluor-1,4-dihydro-7-[3-(4-methoxyphenyl)-1-piperazinyl]-4-oxo-3-chinolincarbonsäure,
6-Chlor-1-cyclopropyl-8-fluor-1,4-dihydro-7-[3-(4-hydroxyphenyl)-1-piperazinyl]-4-oxo-3-chinolincarbonsäure,
8-Chlor-1-cyclopropyl-6-fluor-1,4-dihydro-4-oxo-7-(3-phenyl-1-piperazinyl)-3-chinolincarbonsäure,
8-Chlor-1-cyclopropyl-6-fluor-1,4-dihydro-4-oxo-7-[(4-nitrophenyl)-1-piperazinyl]-3-chinolincarbonsäure,
8-Chlor-1-cyclopropyl-6-fluor-1,4-dihydro-4-oxo-7-[3-(4-piperidinophenyl)-1-piperazinyl]-3-chinolincarbonsäure,
8-Chlor-1-cyclopropyl-6-fluor-1,4-dihydro-4-oxo-7-[3-(3,4-dimethoxyphenyl)-1-piperazinyl]-3-chinolincarbonsäure,
8-Chlor-1-cyclopropyl-6-fluor-1,4-dihydro-4-oxo-7-[3-(3,4,5-trimethoxyphenyl)-1-piperazinyl]-3-chinolincarbonsäure,
8-Chlor-1-cyclopropyl-6-fluor-1,4-dihydro-4-oxo-7-[3-(2-thienyl)-1-piperazinyl]-3-chinolincarbonsäure,
8-Chlor-1-cyclopropyl-6-fluor-1,4-dihydro-4-oxo-7-piperidino-3-chinolincarbonsäure,
7-(3-Amino-1-pyrrolidinyl)-8-chlor-1-cyclopropyl-6-fluor-1,4-dihydro-4-oxo-3-chinolincarbonsäure,
6,8-Dichlor-1-cyclopropyl-1,4-dihydro-4-oxo-7-(1-piperazinyl)-3-chinolincarbonsäure,
7-(4-Acetyl-1-piperazinyl)-6,8-dichlor-1-cyclopropyl-1,4-dihydro-4-oxo-3-chinolincarbonsäure,
7-(4-Acetyl-1-piperazinyl)-6-chlor-1-cyclopropyl-8-fluor-1,4-dihydro-4-oxo-3-chinolincarbonsäure,
6-Chlor-1-cyclopropyl-8-fluor-1,4-dihydro-7-(4-isopropyl-1-piperazinyl)-4-oxo-3-chinolincarbonsäure,
6-Chlor-1-cyclopropyl-8-fluor-1,4-dihydro-4-oxo-7-morpholino-3-chinolincarbonsäure,
6-Chlor-1-cyclopropyl-8-fluor-1,4-dihydro-4-oxo-7-thiomorpholino-3-chinolincarbonsäure,
8-Chlor-1-cyclopropyl-7-(4-ethyl-3-oxo-1-piperazinyl)-6-fluor-1,4-dihydro-4-oxo-3-chinolincarbonsäure.

Die folgenden Beispiele erläutern die Herstellung der Verbindungen gemäß Formel (I') und (II').

**Beispiel A**

6-Chlor-1-cyclopropyl-7,8-difluor-1,4-dihydro-4-oxo-3-chinolincarbonsäure

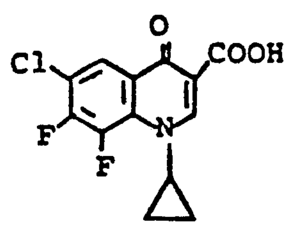

15,7 g (0,65 mol) Magnesiumspäne werden in 40 ml Ethanol und 2 ml Tetrachlormethan verrührt und nach begonnener Reaktion bei 50 - 60° tropfenweise mit 103 g (0,64 Mol) Malonsäurediethylester in 80 ml Ethanol und 250 ml Toluol versetzt. Man rührt 1 Stunde bei dieser Temperatur nach, kühlt auf -5 bis -10°, tropft eine Lösung von 138 g (0,65 mol) 5-Chlor-2,3,4-trifluor-benzoylfluorid in 63 ml Toluol zu, rührt noch 1 Stunde bei 0° und läßt über Nacht bei Raumtemperatur stehen. Danach wird noch 2 Stunden auf 40 - 50° erwärmt, abgekühlt und mit 250 ml Eiswasser und 38,5 ml konzentrierter Schwefelsäure versetzt. Die organische Phase wird abgetrennt, die wäßrige Phase mit 2 mal 150 ml Toluol extrahiert, die vereinigten organischen Phasen mit gesättigter Natriumchlorid-Lösung gewaschen, mit Natriumsulfat getrocknet und eingeengt.

Der Rückstand wird mit 200 ml Wasser versetzt (vorteilhaft ist hier die Zugabe von 0,4 g 4-Toluolsulfonsäure) und zur Desethoxycarbonylierung 5 Stunden unter Rückfluß erhitzt. Man extrahiert mit 3 mal 200 ml Dichlormethan, wäscht mit gesättigter Natriumchlorid-Lösung, trocknet mit Natriumsulfat, engt ein und destilliert im Hochvakuum. Man erhält 103 g (56,5 %) (5-Chlor-2,3,4-trifluor-benzoyl)-essigsäure-ethylester mit einem Siedepunkt von 110°/0,9 Torr.

103 g (0,37 mol) des erhaltenen Esters und 83 g (0,56 mol) Orthoameisensäuretriethylester werden mit 95 g Essigsäureanhydrid 2 Stunden auf 150 - 160° erhitzt und anschließend bei 120 - 130° unter Normaldruck, danach

im Hochvakuum eingeengt. Man erhält 115 g (92 % der Theorie) 2-(5-Chlor-2,3,4-trifluor-benzoyl)-3-ethoxyacrylsäure-ethylester als Öl.

84,1 g (0,25 mol) dieser Verbindung werden in 170 ml Ethanol unter Eiskühlung tropfenweise mit 14,8 g (0,26 mol) Cyclopropylamin versetzt und 2 Stunden bei Raumtemperatur gerührt. Danach wird mit 170 ml Wasser verrührt, in Eis gekühlt, der ausgefallene Niederschlag abgesaugt, mit Wasser und wenig Methanol gewaschen und getrocknet. Es werden 47 g (54 %) 2-(5-Chlor-2,3,4-trifluor-benzoyl)-3-cyclopropylamino-acrylsäureethylester vom Schmelzpunkt 71 - 73° erhalten. Nach dem $^1$H-NMR-Spektrum liegt ein cis-trans-Gemisch vor.

47 g (0,14 mol) dieser Verbindung werden in 230 ml Dimethylformamid mit 9,7 g (0,23 mol) Natriumfluorid 2 Stunden auf 160 - 170° erhitzt. Das Reaktionsgemisch wird in 400 ml Eiswasser eingegossen, der Niederschlag abgesaugt, mit Wasser gewaschen und getrocknet. Man isoliert 44 g (99 %) 6-Chlor-1-cyclopropyl-7,8-difluor-1,4-dihydro-4-oxo-3-chinolincarbonsäure-ethylester vom Schmelzpunkt 169 - 172°.

44 g (0,13 mol) des Chinoloncarbonsäureesters werden in 300 ml Eisessig und 179 ml Wasser mit 33 ml konzentrierter Schwefelsäure versetzt und 2 Stunden auf 150°C erhitzt. Das Reaktionsgemisch wird in 400 ml Eiswasser eingerührt, der Niederschlag abgesaugt, mit Wasser gewaschen und getrocknet. Es werden 37 g (95 % der Theorie) 6-Chlor-1-cyclopropyl-7,8-difluor-1,4-dihydro-4-oxo-3-chinolincarbonsäure mit einem Schmelzpunkt von 200 - 204° isoliert.

**Beispiel B**

8-Chlor-1-cyclopropyl-6,7-difluor-1,4-dihydro-4-oxo-3-chinolincarbonsäure

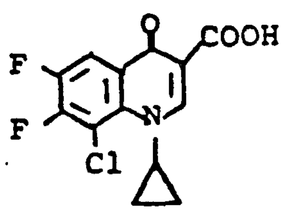

Man setzt 3-Chlor-2,4,5-trifluor-benzoylchlorid analog Beispiel A um, wobei folgende Stufen durchlaufen werden:

(3-Chlor-2,4,5-trifluor-benzoyl)-essigsäure-ethylester als Enol (Ausbeute: 42 %, Schmelzpunkt 72 - 75°),
2-(3-Chlor-2,4,5-trifluor-benzoyl)-3-ethoxy-acrylsäure-ethylester (Rohausbeute: 95 % Öl),
2-(3-Chlor-2,4,5-trifluor-benzoyl)-3-cyclopropylaminoacrylsäureethylester (Ausbeute: 67 %, Schmelzpunkt 78 - 80°),
8-Chlor-1-cyclopropyl-6,7-difluor-1,4-dihydro-4-oxo-3-chinolincarbonsäure-ethylester (Ausbeute: 85 %, Schmelzpunkt 154 - 157°),
8-Chlor-1-cyclopropyl-6,7-difluor-1,4-dihydro-4-oxo-3-chinolincarbonsäure (Ausbeute: 97,6 %, Schmelzpunkt 189 - 192°).

**Beispiel C**

6,8-Dichlor-1-cyclopropyl-7-fluor-1,4-dihydro-4-oxo-3-chinolincarbonsäure

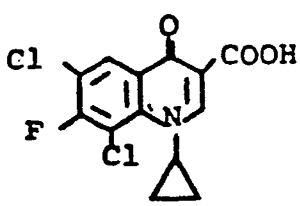

Man setzt 3,5-Dichlor-2,4-difluor-benzoylfluorid analog Beispiel A um, wobei folgende Stufen durchlaufen werden:

(3,5-Dichlor-2,4-difluor-benzoyl)-essigsäure-ethylester (Ausbeute: 43 %, Siedepunkt 133°/2,5 Torr),
2-(3,5-Dichlor-2,4-difluor-benzoyl)-3-ethoxy-acrylsäure-ethylester (Rohausbeute: 91 % Öl),
2-(3,5-Dichlor-2,4-difluor-benzoyl)-3-cyclopropyl-aminoacrylsäureethylester (Ausbeute: 96 %, Schmelzpunkt 71 - 74°),

6,8-Dichlor-1-cyclopropyl-7-fluor-1,4-dihydro-4-oxo-3-chinolincarbonsäure-ethylester (Ausbeute: 97 %, Schmelzpunkt 215 - 217° unter Zersetzung),

6,8-Dichlor-1-cyclopropyl-7-fluor-1,4-dihydro-4-oxo-3-chinolincarbonsäure (Ausbeute: 93 %, Schmelzpunkt 204 - 206°).

**Beispiel 1**

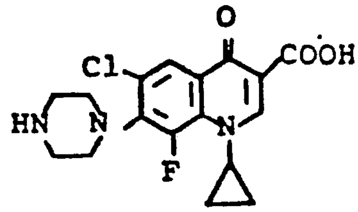

12 g (40 mmol) des Produktes aus Beispiel A werden in 100 ml Pyridin mit 17,2 g (0,2 mol) Piperazin 5 Stunden unter Rückfluß erhitzt. Das Gemisch wird im Vakuum eingeengt, mit 120 ml Wasser verrührt und mit 2 n Salzsäure auf pH 5 eingestellt. Der Niederschlag wird abgesaugt, mit Wasser und Methanol gewaschen, in 80 ml Methanol aufgekocht und getrocknet. Man erhält 12,3 g (84 % der Theorie) 6-Chlor-1-cyclopropyl-8-fluor-1,4-dihydro-4-oxo-7-(1-piperazinyl)-3-chinolincarbonsäure vom Schmelzpunkt 295 - 298° (unter Zersetzung).

Analog Beispiel 1 erhält man folgende in 7-Stellung substituierte 6-Chlor-1-cyclopropyl-8-fluor-1,4-dihydro-4-oxo-3-chinolincarbonsäuren:

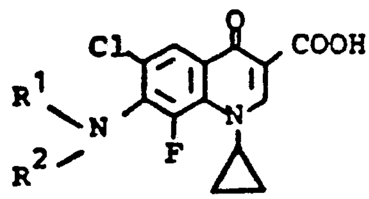

| Beispiel | 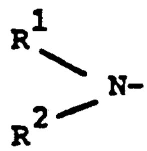 | Schmelzpunkt |
|---|---|---|
| 2 | 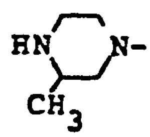 | 258 - 282° (Zersetzung) |
| 3 | 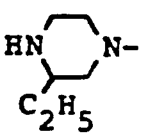 | 191 - 195° (Zersetzung) |
| 4 | 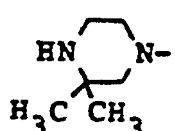 | ab ~ 274° (Zersetzung) |
| 5 | 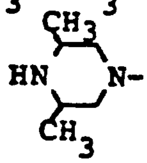 | 255 - 261° (Zersetzung) |
| 6 | 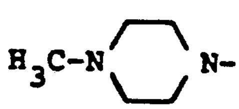 ·HCl | > 320° (Zersetzung) |

| | | |
|---|---|---|
| 7 | 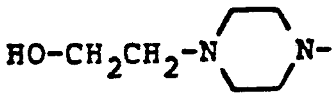 | 276 - 280° (Zersetzung) |
| 8 | 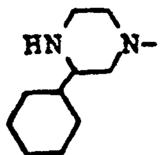 | ab ~ 190° (Zersetzung) |
| 9 | 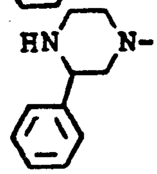 | 154 - 158° |

**Beispiel 10**

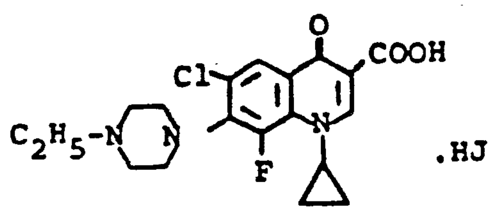

1,83 g (5 mmol) des Produktes aus Beispiel 1 werden in 20 ml Dimethylformamid mit 1,6 g Ethyljodid und 1 g Triethylamin 3 Stunden auf 80° erhitzt. Das Reaktionsgemisch wird im Vakuum eingeengt, der Rückstand mit 20 ml Wasser verrührt und aus Methanol umkristallisiert. Man erhält 0,4 g (15 % der Theorie) 6-Chlor-1-cyclopropyl-7-(4-ethyl-1-piperazinyl)-8-fluor-1,4-dihydro-4-oxo-3-chinolincarbonsäure vom Schmelzpunkt 237 - 242° (unter Zersetzung).

**Beispiel 11**

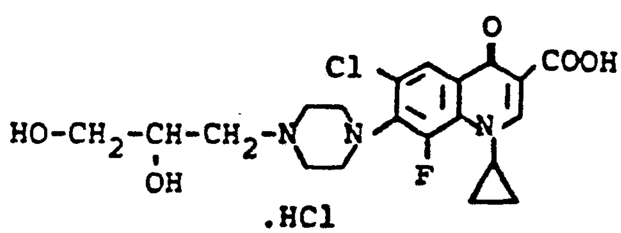

3,65 g (0,01 mmol) des Produktes aus Beispiel 1 werden in 150 ml Ethanol und 30 ml Wasser suspendiert, die Suspension wird mit Essigsäure auf pH 4,6 eingestellt und anschließend bei Raumtemperatur mit 3,4 g (0,02 mol) 2,3-Cyclohexylidenglycerinaldehyd und portionsweise mit 950 mg Natriumcyanoborhydrid versetzt. Man rührt über Nacht bei Raumtemperatur, stellt mit Natriumhydrogencarbonat auf pH 8, extrahiert mit Dichlormethan und engt den Extrakt ein.

Der Rückstand wird in 25 ml Ethanol und 25 ml Wasser mit 3 ml konzentrierter Salzsäure versetzt und 6 Stunden unter Rückfluß erhitzt. Das Gemisch wird eingeengt, in Wasser gelöst, mit Dichlormethan extrahiert, nochmals eingeengt, der Rückstand mit Ethanol verrührt und getrocknet. Man erhält 1,3 g 6-Chlor-1-cyclopropyl-8-fluor-1,4-dihydro-7-[4-(2,3-dihydroxypropyl)-1-piperazinyl]-4-oxo-3-chinolincarbonsäure-Hydrochlorid vom Schmelzpunkt 263 - 266° (unter Zersetzung).

**Beispiel 12**

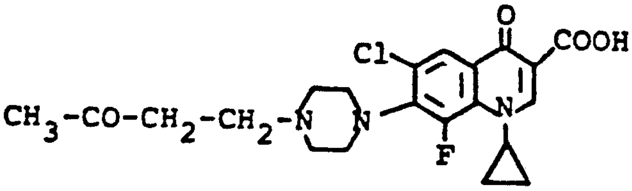

1,83 g (5 mmol) des Produktes aus Beispiel 1 und 1,95 g (28 mmol) Methylvinylketon werden in 25 ml Ethanol 6 Stunden unter Rückfluß erhitzt, der Niederschlag abgesaugt und aus Glykolmonomethylether umkristallisiert. Es werden 1 g (46 % der Theorie) 6-Chlor-1-cyclopropyl-8-fluor-1,4-dihydro-4-oxo-7-[4-(3-oxo-butyl)-1-piperazinyl]-3-chinolincarbonsäure vom Schmelzpunkt 187 - 190° (unter Zersetzung) erhalten.

**Beispiel 13**

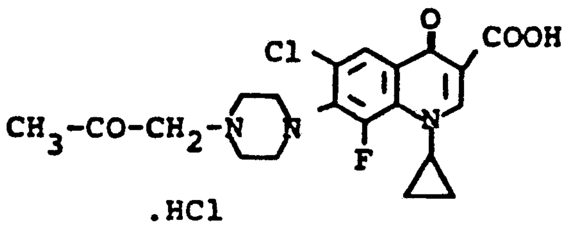

1,83 g (5 mmol) des Produktes aus Beispiel 1 werden in 25 ml Ethanol mit 1,95 g (20 mmol) Chloraceton 6 Stunden unter Rückfluß erhitzt. Man kühlt die Suspension ab, saugt den Niederschlag ab, wäscht gut mit Ethanol und trocknet im Vakuum, wobei 1 g (44 % der Theorie) 6-Chlor-1-cyclopropyl-8-fluor-1,4-dihydro-4-oxo-7-[4-(2-oxo-propyl)-1-piperazinyl]-3-chinolincarbonsäure-Hydrochlorid vom Schmelzpunkt ~ 320°C (unter Zersetzung) erhalten werden.

**Beispiel 14**

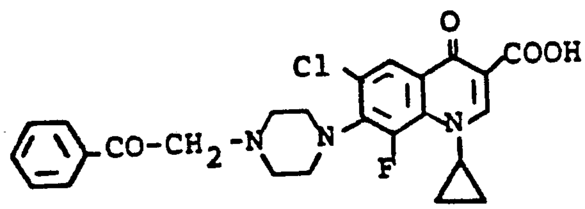

3,66 g (0,01 mol) des Produktes aus Beispiel 1 werden in 50 ml Dimethylformamid mit 2,2 g ω-Chloracetophenon und 2,2 g Triethylamin 10 Stunden auf 60° erhitzt. Das Reaktionsgemisch wird im Vakuum eingeengt, mit 30 ml Wasser verrührt, der Niederschlag abgesaugt, mit Wasser gewaschen und aus Aceton umkristallisiert. Man erhält 1,2 g (25 % der Theorie) 6-Chlor-1-cyclopropyl-8-fluor-1,4-dihydro-4-oxo-7-[4-(2-oxo-2-phenyl-ethyl)-1-piperazinyl]-3-chinolincarbonsäure vom Schmelzpunkt 175 - 179° (unter Zersetzung).

**Beispiel 15**

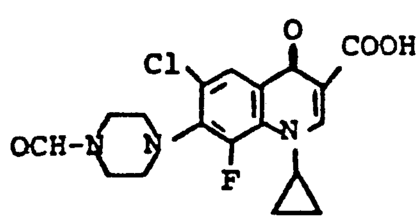

1,5 g (4 mmol) des Produktes aus Beispiel 1 werden in einer Mischung aus 10 ml Dioxan und 170 mg Natriumhydroxid in 2,5 ml Wasser gelöst und dann gleichzeitig mit einer Lösung von 0,7 g Ameisensäureessigsäureanhydrid in 5 ml Dioxan und einer Lösung von 340 mg Natriumhydroxid in 5 ml Wasser versetzt. Man rührt 2 Stunden bei Raumtemperatur, verdünnt mit 30 ml Wasser, saugt den Niederschlag ab, wäscht mit Wasser und Methanol und kristallisiert aus Glykolmonomethylether um. Es werden 0,6 g (38 %) 6-Chlor-1-cyclopropyl-8-fluor-7-(4-formyl-1-piperazinyl)-1,4-dihydro-4-oxo-3-chinolincarbonsäure vom Schmelzpunkt 277 - 278° (unter Zersetzung) erhalten.

**Beispiel 16**

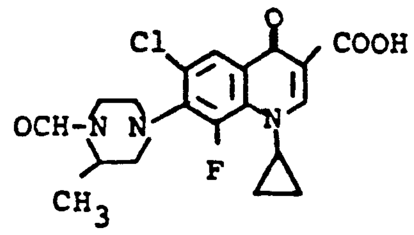

Setzt man analog Beispiel 15 das Produkt aus Beispiel 2 um, dann erhält man 6-Chlor-1-cyclopropyl-8-fluor-7-(4-formyl-3-methyl-1-piperazinyl)-1,4-dihydro-4-oxo-3-chinolincarbonsäure vom Schmelzpunkt 280 - 282° (unter Zersetzung).

**Beispiel 17**

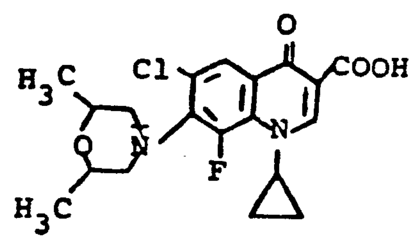

3 g (10 mmol) des Produktes aus Beispiel A werden in 35 ml Dimethylsulfoxid mit 1,2 g (10 mmol) 2,6-Dimethylmorpholin und 2,2 g (20 mmol) Diazabicyclo[2.2.2]octan 5 Stunden auf 140° erhitzt. Man engt im Hochvakuum ein, verrührt mit 30 ml Wasser, stellt mit 2 n Salzsäure auf pH 6, saugt den Niederschlag ab und kristallisiert aus Glykolmonomethylether um. Es werden 1,6 g (41 % der Theorie) 6-Chlor-1-cyclopropyl-8-fluor-1,4-dihydro-7-(2,6-dimethyl-4-morpholinyl)-4-oxo-3-chinolincarbonsäure vom Schmelzpunkt 258 - 261° (unter Zersetzung) erhalten.

**Beispiel 18**

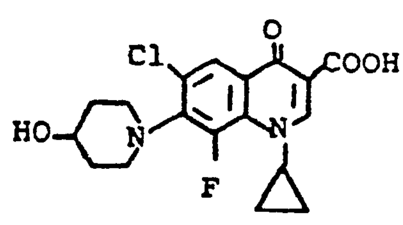

Setzt man analog Beispiel 17 das Produkt aus Beispiel A mit 4-Hydroxypiperidin um, dann erhält man 6-Chlor-1-cyclopropyl-8-fluor-1,4-dihydro-7-(4-hydroxy-1-piperidinyl)-4-oxo-3-chinolincarbonsäure vom Schmelzpunkt 226 - 231° (unter Zersetzung).

**Beispiel 19**

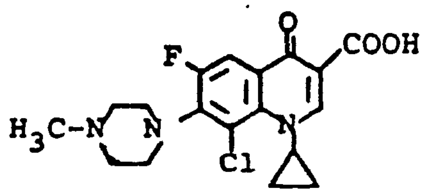

3 g (0,01 mol) des Produktes aus Beispiel B werden in 25 ml Pyridin mit 4 g (0,04 mol) 1-Methyl-piperazin 5 Stunden unter Rückfluß erhitzt. Man engt im Vakuum ein, versetzt mit 20 ml Wasser, stellt mit 2 n Salzsäure auf pH 5 und kristallisiert den ausgefallenen Niederschlag aus Methanol um. Es werden 0,6 g (16 % der Theorie) 8-Chlor-1-cyclopropyl-6-fluor-1,4-dihydro-7-(4-methyl-1-piperazinyl)-4-oxo-3-chinolincarbonsäure vom Schmelzpunkt 293 - 297° (unter Zersetzung) erhalten.

**Beispiel 20**

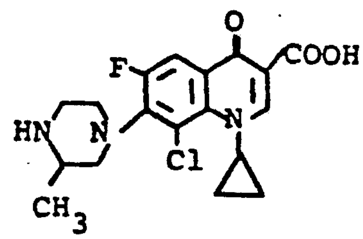

Analog Beispiel 19 erhält man mit 2-Methyl-piperazin 8-Chlor-1-cyclopropyl-6-fluor-1,4-dihydro-7-(3-methyl-1-piperazinyl)-4-oxo-3-chinolincarbonsäure vom Schmelzpunkt 318 - 325° (unter Zersetzung).

**Beispiel 21**

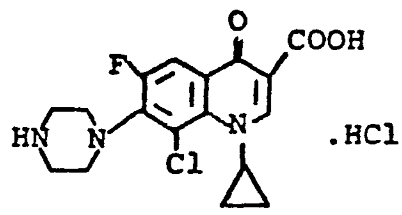

Man setzt analog Beispiel 19 das Produkt aus Beispiel B mit Piperazin 1,5 Stunden unter Rückfluß um und behandelt das Reaktionsgemisch mit Salzsäure, wobei 8-Chlor-1-cyclopropyl-6-fluor-1,4-dihydro-7-(1-piperazinyl)-3-chinolincarbonsäure-Hydrochlorid mit einem Zersetzungspunkt oberhalb 330° erhalten wird.

**Beispiel 22**

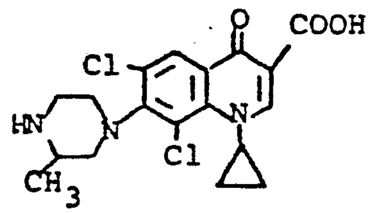

Setzt man das Produkt aus Beispiel C analog Beispiel 19 mit 2-Methyl-piperazin um, dann erhält man 6,8-Dichlor-1-cyclopropyl-1,4-dihydro-7-(3-methyl-1-piperazinyl)-4-oxo-3-chinolincarbonsäure vom Schmelzpunkt 288 - 291° (unter Zersetzung).

**Beispiel 23**

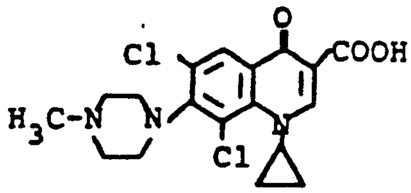

.HCl

3,2 g (0,01 mol) des Produktes aus Beispiel C werden mit 4 g (0,04 mol) 1-Methyl-piperazin 3 Tage auf 80° erhitzt, das Reaktionsgemisch im Vakuum eingeengt, der Rückstand in etwas Wasser aufgenommen und mit 2 n Salzsäure auf pH 7 eingestellt. Beim Stehen in Eis erfolgt Kristallisation. Der Niederschlag wurde abgesaugt und aus Wasser unter Zusatz von etwas Salzsäure umkristallisiert. Man erhält 0,6 g 6,8-Dichlor-1-cyclopropyl-1,4-dihydro-7-(4-methyl-1-piperazinyl)-4-oxo-3-chinolincarbonsäure vom Schmelzpunkt > 300°.

**Beispiel 24**

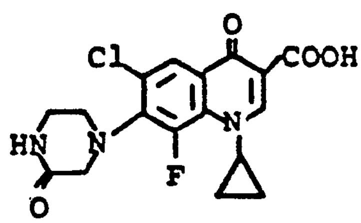

3 g (10 mmol) des Produktes aus Beispiel 1 werden in 25 ml Dimethylsulfoxid mit 1,8 g (18 mmol) 2-Piperazinon und 2,2 g (20 mmol) Diazabicyclo[2.2.2]octan 2 Stunden auf 130° erhitzt. Die Suspension wird mit 2 n Salzsäure auf pH 5 eingestellt, mit 25 ml Wasser versetzt und der Niederschlag abgesaugt, mit 20 ml Methanol ausgekocht und getrocknet. Es werden 1,5 g (39 % der Theorie) 6-Chlor-1-cyclopropyl-8-fluor-1,4-dihydro-4-oxo-7-(3-oxo-1-piperazinyl)-3-chinolincarbonsäure vom Schmelzpunkt 288 - 291° (unter Zersetzung) erhalten.

Die folgenden Beispiele erläutern die Herstellung der erfindungsgemäßen Verbindungen:

**Beispiel 25**

2,4,5-Trifluorbenznylfluorid

Aus dem 2,4,5-Trichlorbenzoylchlorid (Kp. 113/14 mbar, $n^{20}_D$ : 1,5722) wird durch Behandlung mit einem 4-fachen Überschuß wasserfreier HF bei 120°C im Autoklaven während 3 Stunden unter Abspaltung von Chlorwasserstoff das 2,4-Dichlor-5-fluorbenzoylfluorid vom Kp. 98°C/15 mbar, $n^{20}_D$ : 1,5355 hergestellt.

878 g 2,4-Dichlor-5-fluorbenzoylfluorid werden in einem Dreihalskolben mit Rührer, Thermometer und einer

Destillationskolonne in 2,350 ml Tetramethylensulfon mit 1,142 g wasserfreiem KF über 3,5 Stunden bei 200°C umgesetzt. Dabei wird laufend Destillat über die Destillationskolonne abgenommen, anfänglich bei 750 mbar, gegen Ende bei ca. 80 mbar. Das Rohdestillat wird einer fraktionierten Redestillation unterworfen. Man erhält 486 g (65,5 % der Theorie) 2,4,5-Trifluorbenzoylfluorid vom Kp. 52 - 3°/20 mbar, $n^{20}_D$ : 1,4530.

Nach Hydrolyse von 161 g 2,4,5-Trifluorbenzoylfluorid mit einer Lösung von 100 g Natriumhydroxid in 1 l Wasser bei 40° (exotherm bis 70°, Verdünnen mit 1 l Wasser, Ansäuern mit Salzsäure, Filtration und Trocknen erhält man daraus praktisch quantitativ 2,4,5-Trifluorbenzoesäure vom Fp. 95°C.

**Beispiel 26**

Fluorierung von 2,3,4,5-Tetrachlorbenzoylchlorid

Aus 2,994 g 2,3,4,5-Tetrachlorbenzoylchlorid vom Kp. 118°C/0,5 mbar, Fp. 38°C, werden nach Fluorierung mit 1,6 l wasserfreier Fluorwasserstoffsäure in 2 l Methylenchlorid bei 60°C im Autoklaven (5 Stunden) nach Destillation der HF und des Lösungsmittels 2,749 g 2,3,4,5-Tetrachlorbenzoylfluorid vom Fp. 52 - 3°C erhalten.

Diese Menge wird zusammen mit 2,914 g wasserfreiem KF in 7,250 ml Tetramethylensulfon in einem Dreihalskolben erwärmt und zunächst zur Entfernung von eventuellem Restwasser ca. 500 ml des Lösungsmittels wieder abdestilliert. Dann wird das Reaktionsgemisch etwa 4,5 Stunden unter kräftigem Rühren auf 240°C erwärmt. Der Druck von anfänglich 800 mbar wird dabei kontinuierlich bis auf 500 mbar gesenkt. Gleichzeitig nimmt man über die Fraktionierkolonne das Gemisch der Fluorierungsprodukte als Destillat ab. Insgesamt erhält man 1,840 g. Durch fraktionierte Redestillation erhält man daraus:

206 g 2,3,4,5-Tetrafluorbenzoylfluorid,
Kp: 45 - 70°C/20 mbar, $n^{20}_D$ : 1,4372;
954 g 5-Chlor-2,3,4-trifluorbenzoylfluorid,
Kp: 68 - 70°C/20 mbar, $n^{20}_D$ : 1,4764;
330 g 3,5-Dichlor-2,4-difluorbenzoylfluorid
Kp: 97°C/20 mbar, $n^{20}_D$ : 1,5148.

64 g 5-Chlor-2,3,4-trifluorbenzoylfluorid werden mit 13 g $SiCl_4$ in Gegenwart von 0,1 g $AlCl_3$ erwärmt. Die bei etwa 35°C einsetzende Reaktion wird bis zu einer Temperatur von etwa 100°C zu Ende geführt und dann der Rückstand durch Destillation aufgearbeitet. Man erhält:

62 g 5-Chlor-2,3,4-trifluorbenzoylchlorid,
Kp: 88°C/14 mbar, $n^{20}_D$ : 1,5146.

Entsprechend erhält man auch das 3,5-Dichlor-2,4-difluorbenzoylchlorid als Flüssigkeit vom Kp. 113 - 4°C/15 mbar, $n^{20}_D$ : 1,5512.

Wird 5-Chlor-2,3,4-trifluorbenzoylfluorid kurz mit wäßriger Natronlauge behandelt, so erhält man näch Ansäuern und Trocknen Kristalle von 5-Chlor-2,3,4-trifluorbenzoesäure, Fp. 123 - 4°C.

Entsprechend erhält man 3,5-Dichlor-2,4-difluorbenzoesäure, Fp. 179°C.

**Beispiel 27**

5-Chlor-2,3,4-trifluorbenzylalkohol

62 g $NaBH_4$ werden in 320 ml Dioxan vorgelegt. Bei Rückflußtemperatur läßt man hierzu im Verlauf von 6 Stunden die Lösung von 319 g 5-Chlor-2,3,4-trifluorbenzoylfluorid in 640 ml Dioxan zulaufen. Nach einer weiteren Stunde Kochen am Rückfluß wird das Gemisch auf Eis gegeben, mit verdünnter Salzsäure auf pH 1 gestellt, die organische Phase mit Methylenchlorid extrahiert und anschließend destilliert. Man erhält 261 g 2,3,4-Trifluor-5-chlorbenzylalkohol vom Kp. 109°C/12 mbar.

3,5-Dichlor-2,4-difluorbenzylalkohol erhält man entsprechend aus 3,5-Dichlor-2,4-difluorbenzoylfluorid als Kristalle vom Kp. 134°C/13 mbar, Fp. 55°C.

**Beispiel 28**

3-Chlor-2,4,5-trifluorbenzoylfluorid

150 g 2,4,5-Trifluorbenzoesäure werden in 150 ml Chlorsulfonsäure gelöst und nach Zusatz von 3 g Jod mit gasförmigem Chlor bei 50 bis 60°C chloriert. Es wird bis zu einem Umsatz von ca. 35 bis 50 % des Ausgangsmaterials chloriert und dann die Mischung vorsichtig auf Eis zersetzt.

Das Gemisch der kernhalogenierten Säuren wird abgesaugt und getrocknet (die Reinigung einer Probe durch mehrfaches Umkristallisieren ergibt für 3-Chlor-2,4,5-trifluorbenzoesäure einen Fp. 114 - 5°C).

Der Rohansatz wird mit überschüssigem Thionylchlorid in Gegenwart von einigen Tropfen Dimethylformamid in das Gemisch der Säurechloride überführt. Durch Feindestillation einer Probe wird 3-Chlor-2,4,5-trifluorbenzoylchlorid isoliert, Kp. 94°C/18 mbar, $n^{20}_D$ : 1,5164.

Der übrige Teil wird in einem Autoklaven aus rostfreiem Stahl bei ca. -5°C mit 100 ml wasserfreier HF versetzt und nach Abklingen der HCl-Entwicklung zur Beendigung der Reaktion noch kurz bis auf 60°C geheizt und dann durch Destillation aufgearbeitet. Man isoliert: 38 g 3-Chlor-2,4,5-trifluorbenzoylfluorid vom Kp. 65°C/18 mbar, $n^{20}_D$ : 1,4760.

Durch Reduktion des Säurefluorids mit $NaBH_4$ erhält man 2,4,5-Trifluor-3-chlorbenzylalkohol: Kp. 109°C/14 mbar, Fp. 32°C.

39 g 3-Chlor-2,4,5-trifluorbenzylalkohol werden in 92 ml Aceton mit einer Lösung von 20 g $Na_2Cr_2O_7$ in 81 ml Wasser und 14 ml konzentrierter Schwefelsäure während 2 Stunden bei 20°C bis 25°C oxydiert, die organische Phase mit wäßriger Natriumcarbonatlösung gewaschen und mit Natriumsulfat getrocknet. Nach Abdampfen des Lösungsmittels und Destillation erhält man 2,4,5-Trifluor-3-chlorbenzaldehyd, Kp. 72°C/12 mbar, $n^{20}_D$ : 1,5055, langsam kristallisierend; Fp. 31 - 2°C.

**Beispiel 29**

Fluorierung von 2,3,4,5-Tetrachlorbenzonitril

2,3,4,5-Tetrachlorbenzonitril vom F: 123 - 5°C (hergestellt aus 2,3,4,5-Tetrachlorbenzoylchlorid, F: 30° über 2,3,4,5-Tetrachlorbenzoesäureamid, F: 206°) werden mit KF in Tetramethylensulfon fluoriert. Durch fraktionierte Destillation des Fluorierungsansatzes werden isoliert:

2,3,4,5-Tetrafluorbenzonitril, Kp. 59°C/15 mbar, $n^{20}_D$ : 1,4562;
5-Chlor-2,3,4-trifluorbenzonitril, Kp. 78°C/14 mbar, $n^{20}_D$ : 1,4960; und
3,5-Dichlor-2,4-difluorbenzonitril, Kp. 113°C/19 mbar, F: 39 - 40°C.

**Beispiel 30**

3,5-Dichlor-2,4-difluor-benzoesäureamid

Man legt 620 ml konzentrierte wäßrige Ammoniak-Lösung und 600 ml Wasser vor, tropft bei 40 - 50° 458 g (2 mol)

3,5-Dichlor-2,4-difluorbenzoylfluorid zu und rührt anschließend 30 Minuten bei 50° nach. Der Niederschlag wird abgesaugt, mit Wasser gewaschen und getrocknet. Man erhält 408 g (90 % der Theorie) 3,5-Dichlor-2,4-difluorbenzoesäureamid vom Schmelzpunkt 163 - 164°.

Analog erhält man:

5-Chlor-2,3,4-trifluor-benzoesäureamid, Fp. 135 - 137°,
3-Chlor-2,4,5-trifluor-benzoesäureamid, Fp. 125°,
2,4,5-Trifluor-benzoesäureamid, Fp. 145 - 147°,
2,3,4,5-Trichlor-benzoesäureamid, Fp. 206°.

**Beispiel 31**

3,5-Dichlor-2,4-difluor-benzylchlorid

Es werden 354 ml Thionylchlorid und 1 Tropfen Dimethylformamid bei Raumtemperatur vorgelegt und 375 g (1,76 mol) 3,5-Dichlor-2,4-difluor-benzylalkohol zugetropft. Man erhitzt solange unter Rückfluß bis die Gasentwicklung beendet ist. Das überschüssige Thionylchlorid wird abdestilliert und der Rückstand im Vakuum destilliert. Man erhält 386 g (95 % der Theorie) 3,5-Dichlor-2,4-difluorbenzylchlorid vom Schmelzpunkt 107°/12 mbar; $n^{20}_D$ : 1,5368.

Analog erhält man:

5-Chlor-2,3,4-trifluor-benzylchlorid, Kp. 78°/13 mbar; $n^{20}_D$ : 1,4972
3-Chlor-2,4,5-trifluor-benzylchlorid, Kp. 80°/16 mbar; $n^{20}_D$ : 1,4966

**Beispiel 32**

Chlorierung von 5-Chlor-2,3,4-trifluor-benzylchlorid

187 g (0,87 mol) 5-Chlor-2,3,4-trifluor-benzylchlorid werden vorgelegt und bei 100 - 105° unter UV-Bestrahlung mit einem Unterschuß Chlor chloriert. Das Rohgemisch enthält nach gaschromatographischer Bestimmung 66 % 5-Chlor-2,3,4-trifluor-benzalchlorid und 32 % 5-Chlor-2,3,4-trifluor-benzotrichlorid. Nach fraktionierter Destillation erhält man:

94 g (43 % der Theorie) 5-Chlor-2,3,4-trifluor-benzalchlorid, Kp. 88°/12 mbar, $n^{20}_D$ : 1,5082;
33 g (13 % der Theorie) 5-Chlor-2,3,4-trifluor-benzotrichlorid, Kp. 104°/12 mbar, $n^{20}_D$ : 1,5235;

Analog erhält man:
3,5-Dichlor-2,4-difluor-benzalchlorid, Kp. 114°/14 mbar, $n^{20}_D$ : 1,5443,
3,5-Dichlor-2,4-difluor-benzotrichlorid, Kp. 128°/14 mbar, Fp. 43°.

**Beispiel 33**

5-Chlor-2,3,4-trifluor-benzaldehyd

72 g (0,29 mol) 5-Chlor-2,3,4-trifluor-benzalchlorid werden bei 40° zu 220 g 95 %-iger Schwefelsäure gegeben und solange bei 40° nachgerührt, bis die Gasentwicklung beendet ist.

Der Rückstand wird auf Eis gegeben, mit Methylenchlorid extrahiert, die organische Phase mit Natriumsulfat getrocknet, eingeengt und destilliert. Man erhält 18 g (32 % der Theorie) 5-Chlor-2,3,4-trifluor-benzaldehyd vom Siedepunkt 73°/15 mbar, $n^{20}_D$ : 1,5020.

Analog erhält man:
3,5-Dichlor-2,4-difluor-benzaldehyd, Kp. 98°/14 mbar, Fp. 32°C.

**Beispiel für Tabletten enthaltend Wirkstoffe, die mit den Endprodukten der Formel (I') erhalten werden.**

Jede Tablette enthält:

| | |
|---|---|
| Verbindung des Beispiels 1 | 583,0 mg |
| Mikrokristalline Cellulose | 55,0 mg |
| Maisstärke | 72,0 mg |
| Poly-(1-vinyl-2-pyrrolidon) unlöslich | 30,0 mg |
| Hochdisperses Siliciumdioxid | 5,0 mg |
| Magnesiumstearat | 5,0 mg |
| | 750,0 mg |

Die Lackhülle enthält:

| | |
|---|---|
| Poly-(O-hydroxypropyl-O-methyl)-cellulose 15 cp | 6,0 mg |
| Macrogol 4000 rec. INN (Polyethylenglykole DAB) | 2,0 mg |
| Titan-(IV)-oxid | 2,0 mg |
| | 10,0 mg |

Die Endprodukte zeigen bei geringer Toxizität ein breites antibakterielles Spektrum gegen gram-positive und gram-negative Keime, insbesondere gegen Enterobakteriaceen; vor allem auch gegen solche, die resistent sind gegen verschiedene Antibiotika, wie z. B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z. B. Polymeren, Schmiermitteln, Farben, Fasern, Leder, Papier und Holz, von Lebensmitteln und von Wasser.

Die Endprodukte sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die Endprodukte gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Micrococcaceae, wie Staphylokokken, z. B. Staphylococcus aureus, Staph. Epidermidis, (Staph. = Staphylococcus); Lactobacteriaceae, wie Streptokokken, z. B. Streptococcus pyogenes, α- bzw. β-hämolysierende Streptokokken, nicht-γ-hämolysierende Streptokokken, Enterokokken und Diplococcus pneumoniae (Pneumokokken) (Str. = Streptococcus); Enterobacteriaceae, wie Escherichiae-Bakterien der Escherichia-Gruppe, z. B.

Escherichia coli, Enterobacter-Bakterien, z. B. E. aerogenes, E. cloacae (E. = Enterobacter), Klebsiella-Bakterien, z. B. K. pneumoniae (K. = Klebsiella), Serratia, z. B. Serratia marcescens, Proteae-Bakterien der Proteus-Gruppe: Proteus, z. B. Pr. vulgaris, Pr. morganii, Pr. rettgeri, Pr. mirabilis (Pr. = Proteus); Pseudomonadaceae, wie Pseudomonas-Bakterien, z. B. Ps. aeruginosa (Ps. = Pseudomonas); Bacteroidaceae, wie Bacteroides-Bakterien, z. B. Bacteroides fragilis, Mykoplasmen, z. B. Mykoplasma pneumoniae außerdem

Mykobakterien, z. B. Mykobacterium tuberculosis, Mycobacterium leprae und atypische Mykobakterien.

Die obige Aufzählung von Erregern ist lediglich beispielhaft und keineswegs beschränkend aufzufassen. Als Krankheiten, die durch die erfindungsgemäßen Verbindungen verhindert, gebessert und/oder geheilt werden können, seien beispielsweise genannt:

Otitis; Pharyngitis; Pneumonie; Peritonitis; Pyelonephritis; Cystitis; Endocarditis; Systeminfektionen; Bronchitis; Arthritis; lokale Infektionen, septische Erkrankungen.

Aus den Endprodukten können pharmazeutische Zubereitungen hergestellt werden, die neben nicht-toxischen, inerten pharmazeutisch geeigneten Trägerstoffen weitere Wirkstoffe enthalten.

Es können daraus auch pharmazeutische Zubereitungen in Dosierungseinheiten hergestellt werden. Dies bedeutet, daß die Zubereitung in Form einzelner Teile, z. B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z. B. Stärken, Milchzucker, Rohrzucker, Glukose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumcarbonat, (e) Lösungsverzögerer, z. B. Paraffin und (f) Resorptionsbeschleuniger, z. B. quarternäre Ammoniumverbindungen, (g) Netzmittel, z. B. Cetylalkohol, Glycerinmonostearat, (h) Adsorptionsmittel, z. B. Kaolin und Bentonit und (i) Gleitmittel, z. B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmittel enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z. B. Polyethylenglykole, Fette, z. B. Kakaofett und höhere Ester (z. B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z. B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z. B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z. B. Wasser, Ethylalkohol, Propylenglykol, Suspendiermittel, z. B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbesserte Zusätze, z. B. Pfefferminzöl und Eukalyptusöl und Süßmittel, z. B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den Endprodukten auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Die Wirkstoffe oder die pharmazeutischen Zubereitungen können lokal, oral, parenteral, intraperitoneal und/oder rectal, vorzugsweise oral oder parenteral wie intravenös oder intramuskulär appliziert werden.

Im allgemeinen hat es sich sowohl in der Human- als auch in der Veterinärmedizin als vorteilhaft erwiesen, den oder die Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe, vorzugsweise in Mengen von etwa 1 bis etwa 250, insbesondere 3 bis 60 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die Endprodukte können in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden und dadurch eine Förderung des Wachstums und eine Verbesserung der Verwertung des Futters erreicht werden.

In der nachstehenden Tabelle sind die MHK-Werte einiger der Endprodukte angegeben.

Als Vergleich wurden die entsprechenden MHK-Werte der aus J. Med. Chem. <u>23</u>, 1358 (1980) bekannten 1-Ethyl-6-fluor-1,4-dihydro-4-oxo-7-(1-piperazinyl)-3-chinolincarbonsäure ("Norfloxacin") angegeben, wobei sich zeigt, daß die neuen Endprodukte der bekannten Verbindung überlegen sind.

**MIC** /mcg/ml)

| Stamm | Beispiel Nr. | 1 | 2 | 3 | 5 | 6 | 7 | 8 | 10 |
|---|---|---|---|---|---|---|---|---|---|
| E.coli Neumann | | ⩽0,015 | ⩽0,015 | ⩽0,015 | ⩽0,015 | ⩽0,015 | ⩽0,015 | ./. | ⩽0,015 |
| | T 7 | ⩽0,015 | ⩽0,015 | ⩽0,015 | ⩽0,015 | ⩽0,015 | ⩽0,015 | ⩽0,015 | 0,025 |
| | 445/7 | 4 | 8 | 8 | 8 | 4 | 16 | ./. | 16 |
| Klebsiella | 63 | ⩽0,015 | 0,03 | 0,03 | 0,03 | 0,015 | 0,06 | ./. | 0,125 |
| | 6179 | 0,03 | 0,03 | ⩽0,015 | 0,03 | 0,015 | 0.06 | ./. | 0,06 |
| Proteus | 1017 | ⩽0,015 | ./. | ./. | ./. | 0,06 | 0,125 | ./. | ./. |
| Providencia | 12012 | ⩽0,015 | 0,03 | ./. | ./. | 0,03 | ./. | ./. | ./. |
| | 12052 | 8 | 16 | 16 | 32 | 4 | 16 | ./. | ./. |
| Staph. FK | 422 | 0,25 | 0,25 | 0,25 | 0,25 | 0,025 | 0,5 | 0,06 | ./. |
| | 1756 | 0,25 | 0,5 | 0,25 | 0,5 | 0,5 | 0,5 | 0.06 | ./. |
| | 133 | 0,25 | 0,25 | 0,25 | 0,5 | 0,25 | 0,25 | 0,06 | ./. |
| Pseudom. Ellsworth | | 0,06 | ./. | ./. | ./. | ./. | ./. | ./. | ./. |

Agardilutionstest / Isosensitestmedium

**MIC** (mcg/ml)

| Stamm | Beispiel Nr. | 17 | 12 | 13 | 14 | 17 | 19 | 20 | 21 | 22 | Norfloxacin |
|---|---|---|---|---|---|---|---|---|---|---|---|
| E.coli Neumann | | ./. | ≤0,015 | ≤0,015 | ≤0,015 | ./. | ≤0,015 | ≤0,015 | ≤0,015 | ≤0,015 | 0,06 |
| | T 7 | ./. | ≤0,015 | ≤0,015 | ≤0,015 | ./. | ≤0,015 | ≤0,015 | ≤0,015 | ≤0,015 | 0,03 |
| | 455/7 | ./. | 8 | 8 | 2 | ./. | 1 | 1 | 1 | 2 | 16 |
| Klebsiella | 63 | ./. | ≤0,015 | ≤0,015 | 0,06 | ./. | 0,03 | ≤0,015 | 0,06 | 0,03 | 0,125 |
| | 6179 | ./. | 0,06 | 0,06 | 0,25 | ./. | 0,03 | 0,03 | 0,03 | 0,06 | 0,25 |
| Proteus | 1017 | ./. | 0,03 | ≤0,015 | 0,025 | ./. | 0,03 | ≤0,015 | 0,03 | 0,06 | 0,03 |
| Providencia | 12012 | ./. | ≤0,015 | ≤0,015 | 0,06 | ./. | 0,03 | 0,03 | 0,06 | 0,06 | 0,03 |
| | 12052 | ./. | 16 | 16 | 16 | ./. | 4 | 4 | 1 | 2 | 64 |
| Staph. FK | 422 | ≤0,015 | 0,25 | 0,25 | ./. | ≤0,015 | 0,06 | 0,06 | 0,125 | 0,125 | 0,5 |
| | 1756 | ≤0,015 | 0,25 | 0,25 | ./. | ≤0,015 | 0,06 | 0,06 | 0,125 | 0,125 | 1 |
| | 133 | ./. | 0,25 | 0,25 | ./. | ./. | 0,06 | 0,06 | 0,06 | 0,125 | 0,5 |
| Pseud. Ellsworth | | ./. | 0,125 | 0,125 | ./. | ./. | 0,125 | 0,25 | ./. | ./. | 0,125 |

Agardilutionstest / Isosensitestmedium

**Patentansprüche**

1. 2,4,5-Trihalogen- bzw. 2,3,4,5-Tetrahalogenbenzolderivate der Formel (I)

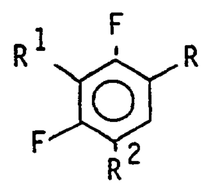

(I)

in welcher
R für -COOH, -COCl, -COF, -CN, -$CONH_2$, -$CH_2OH$, -$CH_2Cl$, -$CHCl_2$, -$CCl_3$ oder -CHO,
$R^1$ für Cl oder F und
$R^2$ für Cl oder F stehen,
wobei nur $R^1$ oder $R^2$ F sein können,
ausgenommen 2,4,5-Trifluorbenzoesäure und 2,4,5-Trifluorbenzoylchlorid.

2. 5-Chlor-2,3,4-trifluorbenzoylfluorid.
3. 3-Chlor-2,4,5-trifluorbenzoylchlorid.
4. 3,5-Dichlor-2,4-difluorbenzoylfluorid.
5. Verfahren zur Herstellung von 2,4,5-Trihalogen- bzw. 2,3,4,5-Tetrahalogenbenzolderivaten der Formel (I)

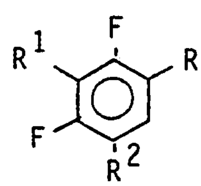

(I)

in welcher
R für -COOH, -COCl, -COF, -CN, -$CONH_2$, -$CH_2OH$, -$CH_2Cl$, -$CHCl_2$ -$CCl_3$ oder -CHO,
$R^1$ für H, Cl oder F und
$R^2$ für Cl oder F stehen,
wobei $R^1$ und $R^2$ nicht gleichzeitig F sein können,
dadurch gekennzeichnet, daß man 2,3,4,5-Tetrachlorbenzonitril mit 1,1 bis 2 Mol Kaliumfluorid pro auszutauschendem Chloratom in einem Lösungsmittel bei erhöhter Temperatur umsetzt und die erhaltenen Nitrile gegebenenfalls in an sich bekannter Weise in die Verbindungen der Formel (I) überführt.

6. Verfahren zur Herstellung von 3-Chlor-2,4,5-trifluorbenzoylchlorid, dadurch gekennzeichnet, daß man 2,4,5-Trifluorbenzoesäure chloriert zur 3-Chlor-2,4,5-trifluorbenzoesäure und diese mit Thionylchlorid umsetzt.

7. Verfahren zur Herstellung von 2,4,5-Trifluorbenzoylfluorid, dadurch gekennzeichnet, daß man 2,4-Dichlor-5-fluorbenzoylchlorid mit 1,1 bis 2 Mol Kaliumfluorid pro auszutauschendem Chloratom in einem Lösungsmittel

bei erhöhter Temperatur umsetzt.

8. Verfahren zur Herstellung von 2,3,4,5-Tetrahalogenbenzolderivaten der Formel (II)

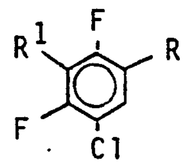

(II)

in welcher

R' für $-COCl$ oder $-COF$ und

$R^1$ für Cl oder F stehen,

dadurch gekennzeichnet, daß man 2,3,4,5-Tetrachlorbenzoylchlorid oder 2,3,4,5-Tetrachlorbenzoylfluorid, gegebenenfalls nach Fluorierung mit Fluorwasserstoffsäure, mit 1,1 bis 2 Mol Kaliumfluorid pro auszutauschendem Chloratom in einem Lösungsmittel bei erhöhter Temperatur umsetzt.

9. Verwendung von 2,4,5-Trihalogen- bzw. 2,3,4,5-Tetrahalogenbenzolderivaten der Formel (I)

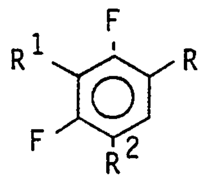

(I)

in welcher

R für $-COOH$, $-COCl$, $-COF$, $-CN$, $-CONH_2$, $-CH_2OH$, $-CH_2Cl$, $-CHCl_2$, $-CCl_3$ oder $-CHO$,

$R^1$ für H, Cl oder F und

$R^2$ für Cl oder F stehen,

wobei $R^1$ und $R^2$ nicht gleichzeitig F sein können,

als Ausgangs- bzw. Zwischenprodukte für die Synthese von Arzneimitteln aus der Gruppe der 7-Amino-1,4-dihydro-4-oxo-chinolincarbonsäuren.

## Claims

1. 2,4,5-Trihalogeno- and 2,3,4,5-tetrahalogenobenzene derivatives of the formula (I)

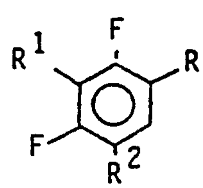

(I)

in which

R represents $-COOH$, $-COCl$, $-COF$, $-CN$, $-CONH_2$, $-CH_2OH$, $-CH_2Cl$, $-CHCl_2$, $-CCl_3$ or $-CHO$,

$R^1$ represents Cl or F, and

$R^2$ represents Cl or F,

it only being possible for $R^1$ or $R^2$ to be F, excluding 2,4,5-trifluorobenzoic acid and 2,4,5-trifluorobenzoyl chloride.

2. 5-Chloro-2,3,4-trifluorobenzoyl fluoride.

3. 3-Chloro-2,4,5-trifluorobenzoyl chloride.

4. 3,5-Dichloro-2,4-difluorobenzoyl fluoride.

5. Process for the preparation of 2,4,5-trihalogeno- and 2,3,4,5-tetrahalogenobenzene derivatives of the formula (I)

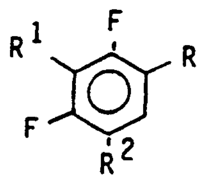

(I)

in which
R represents -COOH, -COCl, -COF, -CN, $-CONH_2$, $-CH_2OH$, $-CH_2Cl$, $-CHCl_2$, $-CCl_3$ or -CHO,
$R^1$ represents H, Cl or F, and
$R^2$ represents Cl or F,
it not being possible for both $R^1$ and $R^2$ to be F simultaneously, characterised in that 2,3,4,5-tetrachlorobenzonitrile is reacted with 1.1 to 2 moles of potassium fluoride per chlorine atom to be exchanged in a solvent at elevated temperature, and the resulting nitriles are, where appropriate, converted in a manner known per se into the compounds of the formula (I).

6. Process for the preparation of 3-chloro-2,4,5-trifluorobenzoyl chloride, characterised in that 2,4,5-trifluorobenzoic acid is chlorinated to give 3-chloro-2,4,5-trifluorobenzoic acid, and the latter is reacted with thionyl chloride.

7. Process for the preparation of 2,4,5-trifluorobenzoyl fluoride, characterised in that 2,4-dichloro-5-fluorobenzoyl chloride is reacted with 1.1 to 2 moles of potassium fluoride per chlorine atom to be exchanged in a solvent at elevated temperature.

8. Process for the preparation of 2,3,4,5-tetrahalogenobenzene derivatives of the formula (II)

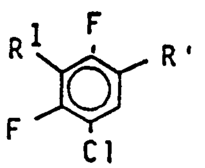

(II)

in which
R' represents -COCl or -COF, and
$R^1$ represents Cl or F,
characterised in that 2,3,4,5-tetrachlorobenzoyl chloride or 2,3,4,5-tetrachlorobenzoyl fluoride is reacted, where appropriate after fluorination with hydrofluoric acid, with 1.1 to 2 moles of potassium fluoride per chlorine atom to be exchanged in a solvent at elevated temperature.

9. Use of 2,4,5-trihalogeno- and 2,3,4,5-tetrahalogenobenzene derivatives of the formula (I)

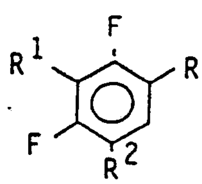

(I)

in which
R represents -COOH, -COCl, -COF, -CN, $-CONH_2$, $-CH_2OH$, $-CH_2Cl$, $-CHCl_2$, $CCl_3$ or -CHO,
$R^1$ represents H, Cl or F, and
$R^2$ represents Cl or F,
it not being possible for $R^1$ and $R^2$ to be F simultaneously, as starting materials or intermediates for the synthesis of medicaments from the group comprising 7-amino-1,4-dihydro-4-oxo-quinolinecarboxylic acids.

**Revendications**

1. Dérivés de 2,4,5-trihalogéno- et 2,3,4,5-tetrahalogéno-benzènes de formule I

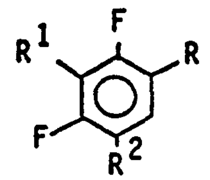

(I)

dans laquelle

R représente -COOH, -COCl, -COF, -CN -$CONH_2$, -$CH_2OH$, -$CH_2Cl$, -$CHCl_2$, -$CCl_3$ ou -CHO,

$R^1$ représente Cl ou F et

$R^2$ représente Cl ou F,

un seul des symboles $R^1$ et $R^2$ pouvant représenter F, à l'exception de l'acide 2,4,5-trifluorobenzoïque et du chlorure de 2,4,5-trifluorobenzoyle.

2. Le fluorure de 5-chloro-2,3,4-trifluorobenzoyle.

3. Le chlorure de 3-chloro-2,4,5-trifluorobenzoyle.

4. Le fluorure de 3,5-dichloro-2,4-difluorobenzoyle.

5. Procédé de préparation des dérivés de 2,4,5-trihalogéno- et 2,3,4,5-tétrahalogéno-benzènes de formule I

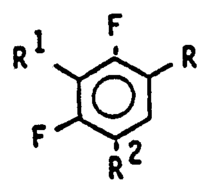

(I)

dans laquelle

R -COOH, -COCl, -COF, -CN, -$CONH_2$, -$CH_2OH$, -$CH_2Cl$, -$CHCl_2$, $CCl_3$ ou -CHO,

$R^1$ représente H, Cl ou F et

$R^2$ représente Cl ou F,

étant spécifié que $R^1$ et $R^2$ ne peuvent représenter tous deux F, caractérisé en ce que l'on fait réagir le 2,3,4,5-tétrachlorobenzonitrile avec 1,1 à 2 mol de fluorure de potassium par atome de chlore à échanger dans un solvant à haute température et on convertit le cas échéant les nitriles obtenus, de manière connue en soi, en les composés de formule I.

6. Procédé de préparation du chlorure de 3-chloro-2,4,5-trifluorobenzoyle, caractérisé en ce que l'on convertit l'acide 2,4,5-trifluorobenzoïque par chloration en l'acide 3-chloro-2,4,5-trifluorobenzoïque et on fait réagir ce dernier avec le chlorure de thionyle.

7. Procédé de préparation du fluorure de 2,4,5-trifluorobenzoyle, caractérisé en ce que l'on fait réagir le chlorure de 2,4-dichloro-5-fluorobenzoyle avec 1,1 à 2 mol de fluorure de potassium par atome de chlore à échanger dans un solvant à température élevée.

8. Procédé de préparation des dérivés de 2,3,4,5-tétrahalogénobenzènes de formule II

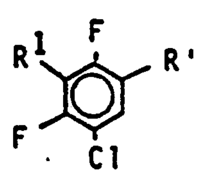

(II)

dans laquelle

R' représente -COCl ou -COF et

$R^1$ représente Cl ou F,

caractérisé en ce que l'on fait réagir le chlorure de 2,3,4,5-tétrachlorobenzoyle ou le fluorure de 2,3,4,5-tétrachlorobenzoyle, éventuellement après fluoration par l'acide fluorhydrique, avec 1,1 à 2 mol de fluorure de potassium par atome de chlore à échanger, dans un solvant et à température élevée.

9. Utilisation des dérivés de 2,4,5-trihalogéno- et 2,3,4,5-tétrahalogéno-benzènes de formule I

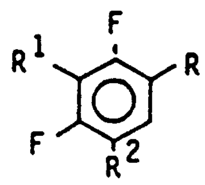

(I)

dans laquelle

R représente -COOH, -COCl, -COF, -CN, $-CONH_2$, $-CH_2OH$, $-CH_2Cl$, $-CHCl_2$, $-CCl_3$ ou -CHO,

$R^1$ représente H, Cl ou F,

$R^2$ représente Cl ou F,

étant spécifié que $R^1$ et $R^2$ ne peuvent représenter tous deux F, en tant que produits de départ ou produits intermédiaires de la synthèse de médicaments du groupe des acides 7-amino-1,4-dihydro-4-oxo-quinoléine-carboxyliques.